# EUROPEAN PATENT SPECIFICATION

(11) **EP 2 931 876 B1**
(45) Date of publication and mention of the grant of the patent: **29.05.2019**
(21) Application number: 13862555.3
(22) Date of filing: 12.12.2013
(51) Int. Cl.: C12N 5/0775, A61K 35/28, A61P 13/12, A61P 9/00, A61K 9/00, A61K 31/7028

(54) **TREATMENT OF DISEASES OF ENDOTHELIAL DYSFUNCTION AND INFLAMMATION**
BEHANDLUNG VON ERKRANKUNGEN AUFGRUND ENDOTHELIALER DYSFUNKTION UND ENTZÜNDUNGEN
TRAITEMENT DE MALADIES DU DYSFONCTIONNEMENT ENDOTHÉLIAL ET DE L'INFLAMMATION ENDOTHÉLIALE

(30) Priority: 12.12.2012 US 201261736361 P
(43) Date of publication of application: 21.10.2015
(73) Proprietor: Mesoblast, Inc., New York, NY 10017 (US)
(72) Inventor: KIMPTON, Wayne Gregory, Croydon North Victoria 3136 (AU); BAILEY, Simon Reeves, Kensington Victoria 3031 (AU); ITESCU, Silviu, Melbourne Victoria 3000 (AU); GHOSH, Peter, Fairlight New South Wales 2094 (AU)
(74) Representative: Schnappauf, Georg
(86) International application number: PCT/AU2013/001452
(87) International publication number: WO 2014/089623

(56) References cited:
- WO-A1-2004/084921
- WO-A1-2004/084921
- WO-A1-2010/005527
- WO-A1-2012/162754
- WO-A1-2012/162754
- WO-A1-2013/003899
- WO-A1-2013/003899
- WO-A2-2006/121445
- WO-A2-2010/138180
- WO-A2-2012/047733
- YAMAZA TAKAYOSHI ET AL: "Immunomodulatory properties of stem cells from human exfoliated deciduous teeth", STEM CELL RESEARCH & THERAPY, BIOMED CENTRAL LTD, LONDON, UK, vol. 1, no. 1, 15 March 2010 (2010-03-15), page 5, XP021070886, ISSN: 1757-6512
- T. L. BONFIELD ET AL: "Human mesenchymal stem cells suppress chronic airway inflammation in the murine ovalbumin asthma model", AMERICAN JOURNAL OF PHYSIOLOGY. LUNG CELLULAR AND MOLECULAR PHYSIOLOGY, vol. 299, no. 6, 1 December 2010 (2010-12-01), pages 760-770, XP055138815, ISSN: 1040-0605, DOI: 10.1152/ajplung.00182.2009
- HONGTING JIN ET AL: "The effects of autologous bone marrow mesenchymal stem cell arterial perfusion on vascular repair and angiogenesis in osteonecrosis of the femoral head in dogs", INTERNATIONAL ORTHOPAEDICS, SPRINGER, BERLIN, DE, vol. 36, no. 12, 13 October 2012 (2012-10-13), pages 2589-2596, XP035145468, ISSN: 1432-5195, DOI: 10.1007/S00264-012-1674-7
- GONZALEZ M A ET AL: "Adipose-Derived Mesenchymal Stem Cells Alleviate Experimental Colitis by Inhibiting Inflammatory and Autoimmune Responses", GASTROENTEROLOGY, ELSEVIER, PHILADELPHIA, PA, vol. 136, no. 3, 1 March 2009 (2009-03-01) , pages 978-989, XP025997941, ISSN: 0016-5085, DOI: 10.1053/J.GASTRO.2008.11.041 [retrieved on 2008-11-27]
- GONZALEZ MANUEL A ET AL: "Treatment of Experimental Arthritis by Inducing Immune Tolerance With Human Adipose-Derived Mesenchymal Stem Cells", ARTHRITIS & RHEUMATISM, WILEY, US, vol. 60, no. 4, 1 April 2009 (2009-04-01), pages 1006-1019, XP009127144, ISSN: 0004-3591, DOI: 10.1002/ART.24405
- GHOSH PETER ET AL: "Pentosan polysulfate promotes proliferation and chondrogenic differentiation of adult human bone marrow-derived mesenchymal precursor cells", ARTHRITIS RESEARCH AND THERAPY, BIOMED CENTRAL, LONDON, GB, vol. 12, no. 1, 18 February 2010 (2010-02-18), page R28, XP021070748, ISSN: 1478-6354
- TACGHEE YI ET AL: "Immunomodulatory properties of mesenchymal stem cells and their therapeutic applications", ARCHIVES OF PHARMACAL RESEARCH, PHARMACEUTICAL SOCIETY OF KOREA, HEIDELBERG, vol. 35, no. 2, 28 February 2012 (2012-02-28), pages 213-221, XP035021439, ISSN: 1976-3786, DOI: 10.1007/S12272-012-0202-Z
- LAURA M. DOOLEY ET AL: "Effect of Mesenchymal Precursor Cells on the Systemic Inflammatory Response and Endothelial Dysfunction in an Ovine Model of Collagen-Induced Arthritis", PLOS ONE, vol. 10, no. 5, 7 May 2015 (2015-05-07), page e0124144, XP055265083, DOI: 10.1371/journal.pone.0124144
- BONFIELD T. L. ET AL.: 'Human mesenchymal stem cells suppress chronic airway inflammation in the murine ovalbumin asthma model' AMERICAN JOURNAL OF PHYSIOLOGY LUNG CELLULAR AND MOLECULAR PHYSIOLOGY vol. 299, 2010, pages L760 - L770, XP055138815
- YAMAZA T. ET AL.: 'Immunomodulatory properties of stem cells from human exfoliated deciduous teeth' STEM CELL RESEARCH AND THERAPY vol. 1, 2010, XP021070886
- Peter N. Van Buren ET AL: "Hypertension in Diabetic Nephropathy: Epidemiology, Mechanisms, and Management", Advances in Chronic Kidney Disease, vol. 18, no. 1, 1 January 2011 (2011-01-01), pages 28-41, XP055432587, USA ISSN: 1548-5595, DOI: 10.1053/j.ackd.2010.10.003

## Description

### FIELD

The present disclosure relates to methods for treating or preventing diseases of endothelial dysfunction or inflammation and conditions associated therewith.

### BACKGROUND

Endothelial dysfunction is characterized by a shift of the actions of the endothelium toward reduced vasodilation, a proinflammatory state, and prothrombic properties. It is the general consensus of vascular biologists that endothelial injury with resulting dysfunction is the initiating event in atherosclerosis (Ross Nature 362:801-9, 1993) and plays an important role in the ischemic manifestations of coronary disease. Endothelial dysfunction also precedes the physical presence of atherosclerosis (Reddy et al., J Am Coll Cardiol 23:833-843, 1994). It is also associated with most forms of cardiovascular disease (such as hypertension, coronary artery disease, chronic heart failure and peripheral artery disease), complications of diabetes (such as nephropathy) and chronic renal failure.

Mechanisms that participate in the reduced vasodilatory responses in endothelial dysfunction include reduced nitric oxide generation, oxidative excess, and reduced production of hyperpolarizing factor. Upregulation of adhesion molecules, generation of chemokines such as macrophage chemoattractant peptide-1, and production of plasminogen activator inhibitor-1 participate in the inflammatory response and contribute to a prothrombic state. Vasoactive peptides such as angiotensin II and endothelin-1; the accumulation of asymmetric dimethylarginine, an endogenous nitric oxide inhibitor; hypercholesterolemia; hyperhomocysteinemia; altered insulin signaling; and hyperglycemia can contribute to these different mechanisms. For example, an excess of angiotensin II is observed in conditions associated with endothelial dysfunction. Angiotensin II is considered a competitive inhibitor of angiotensin I, with angiotenisin I being involved in inhibiting thickening of the endothelium, promoting endothelial survival, stabilization of supporting perivascular cells and inhibition of endothelial permeability. Excessive levels of angiotensin II inhibit these beneficial effects.

Endothelial dysfunction is also an important early event in the pathogenesis of atherosclerosis, contributing to plaque initiation and progression.

Endothelial dysfunction is also a major contributor to the effects of inflammatory diseases such as sepsis. The pathogenesis of sepsis is a result of a complex network of events. Components of the Gram-negative bacterial cell wall (endotoxins) are the predominant (though not exclusive) species responsible for the initiation of sepsis. Endotoxins in addition to other bacterial molecules trigger a generalized response that involves both cellular and humoral pathways with the generation of pro- and anti-inflammatory mediators. These mediators include cytokines, coagulation factors, adhesion molecules, myocardial depressant substances and heat shock proteins. The endothelium is a major target of sepsis-induced events and endothelial cell damage and dysfunction accounts for much of the pathology of septic shock. Vascular endothelial cells are among the first cells in the body that come into contact with circulating bacterial molecules. Endothelial cells possess mechanisms that recognize structural patterns of bacterial pathogens and subsequently initiate the expression of inflammatory mediators.

An approach for treatment of endothelial dysfunction is to address the components in the disease process that trigger dysfunction of the endothelium. For example, decrease of homocysteine levels in hyperhomocysteinemia by supplementation with folic acid can improve endothelial dysfunction. L-Arginine and tetrahydrobiopterin, as well as tetrahydrobiopterin mimetics, may improve endothelial function via increased nitric oxide bioavailability. However, some studies have not found L-arginine administration to improve endothelial dysfunction.

Statins have proven to have beneficial effects on endothelial dysfunction, which may be the result in part of lipid lowering but also of their pleiotropic anti-inflammatory effects. However, statins are associated with muscle pain and rhabdomyolysis (which can lead to kidney failure and death), muscle weakness, neuropathy and memory loss. Furthermore, statins have a relatively short half life and require regular dosing to provide a therapeutic benefit. For example, atorvastatin has an effective half life of about 20-30 hours and is considered a long-acting statin.

Peroxisome proliferator-activated receptor-γ agonists have also been shown to ameliorate endothelial dysfunction. However, the potential benefits of these compounds should be carefully assessed against the safety concerns related to this class of compounds on fluid retention and congestive heart failure as well as the possible enhanced cardiovascular risk.

Yamaza et al., 2010, Stem Cell Research & Therapy 1:5, pages 1-10 disclose the use of mesenchymal stem cells expressing STRO-1 for treating lupus erythematosus, a disease of vascular endothelial dysfunction or inflammation, in a mouse model.

Bonfield et al., 2010, Am J Physiol Lung Cell Mol Physiol 299:760-770 disclose the use of mesenchymal stem cells for treating chronic airway inflammation in a mouse asthma model.

Jin et al., 2012, International Orthopaedics 36:2589-2596 disclose the use of mesenchymal stem cells for vascular repair and angiogenesis in a dog model of osteonecrosis.

Gonzalez et al., 2009, Gastroenterology 136:978-989 disclose the use of adipose-derived stem cells for the treatment of experimental colitis.

Gonzalez et al., 2009, Arthritis & Rheumatism 60:1006-1019 disclose the use of adipose-derived stem cells for the treatment of experimental arthritis.

International Patent Application Publication No. WO 2006/121445 A2 discloses a method for treating acute and chronic renal failure and inflammatory disorders with mesenchymal stem cells.

International Patent Application Publication No. WO 2012/162754 A1 discloses mesenchymal stem cells (MSCs), including STRO-1^{bright} MSCs, for treating an inflammatory disease, e.g., inflammatory encephalomyelitis.

International Patent Application Publication No. WO 2004/084921 A1 discloses the use of bone-derived MSCs, including STRO-1^{bright} MSCs, for inducing neovascularization to treat ischemic/damaged tissues.

International Patent Application Publication No. WO 2013/003899 A1 discloses the use of bone-derived MSCs, including STRO-1bright MSCs, for inducing neovascularization to treat ischemic/damaged tissues.

Ghosh et al., 2010, Arthritis Research & Therapy 12:R28, pages 1 to 17 disclose that pentosan polysulfate promotes proliferation and chondrogenic differentiation of adult human bone marrow-derived mesenchymal precursor cells.

Yi et al., 2012, Arch Pharma Res 35:213-221 reviews the immunomodulatory properties of mesenchymal stem cells and their therapeutic applications.

It will be clear from the foregoing, that there is a need in the art for treating or preventing diseases of endothelial dysfunction or inflammation.

### SUMMARY

The present invention is directed to the subject-matter set forth in the appended claims.

In arriving at the present invention, the inventors showed that administering STRO-1 expressing mesenchymal progenitor cells (MPCs) or progeny thereof systemically (e.g., intravenously) to animals suffering from endothelial dysfunction increased coronary artery response to endothelium-specific dilators (bradykinin and carbachol) but not to the smooth muscle dilator sodium nitroprusside. For example, the MPCs or progeny thereof increased maximal response to the endothelium-specific dilators, which is an indication of reduced endothelial dysfunction compared to results in animals which were not treated with the MPCs or progeny. These data indicate that STRO-1 expressing MPCs or progeny thereof or one or more factors secreted therefrom treat or prevent development of endothelial dysfunction.

Based on the findings of the inventors, the present disclosure provides a method for treating or preventing a disease of vascular endothelial dysfunction or inflammation in a subject, the method comprising systemically administering to the subject a population of cells enriched for STRO-1⁺ cells and/or progeny thereof and/or soluble factors derived therefrom.

In one example, the subject suffers from a condition caused by endothelial dysfunction.

In one example, the endothelial dysfunction is within a blood vessel, e.g., is vascular endothelial dysfunction.

In one example, the condition caused by endothelial dysfunction is selected from the group consisting of sepsis, hypertension, coronary artery disease, chronic heart failure and peripheral artery disease, nephropathy and chronic renal failure.

In one example, the condition caused by endothelial dysfunction is nephropathy. Thus, in one example, the present disclosure provides a method for treating or preventing nephropathy in a subject, the method comprising systemically administering to the subject a population of cells enriched for STRO-1⁺ cells and/or progeny thereof and/or soluble factors derived therefrom.

In one example, the nephropathy is diabetic nephrapothy. Thus, in one example, the present disclosure provides a method for treating or preventing diabetic nephropathy in a subject, the method comprising systemically administering to the subject a population of cells enriched for STRO-1⁺ cells and/or progeny thereof and/or soluble factors derived therefrom.

In another example, the condition is atherosclerosis. Thus, in one example, the present disclosure provides a method for treating or preventing atherosclerosis in a subject, the method comprising systemically administering to the subject a population of cells enriched for STRO-1⁺ cells and/or progeny thereof and/or soluble factors derived therefrom.

In one example, the disease is a disease of inflammation. The disease of inflammation may be a systemic inflammatory response syndrome, such as sepsis, septic shock or a sepsis-like condition. In another example, the condition is sepsis or septic shock. Thus, in one example, the present disclosure provides a method for treating or preventing sepsis or septic shock in a subject, the method comprising systemically administering to the subject a population of cells enriched for STRO-1⁺ cells and/or progeny thereof and/or soluble factors derived therefrom.

The sepsis, septic shock or a sepsis-like condition may be caused by a virus, fungus, protozoan or bacterium.

In one example, the method comprises administering a population of cells enriched for STRO-1^{bright} cells and/or progeny thereof and/or soluble factors derived therefrom.

In one example, the population enriched for STRO-1⁺ cells and/or progeny thereof and/or soluble factors derived therefrom are administered intravenously. In this regard, the inventors have shown that STRO-1⁺ cells and/or progeny thereof administered systemically can improve endothelial dysfunction. Thus, the disclosure contemplates administration of the STRO-1⁺ cells and/or progeny thereof at a site remote from a site of endothelial dysfunction in a subject.

In one example, the STRO-1⁺ cells and/or progeny thereof and/or soluble factors derived therefrom are administered in an amount sufficient to reduce IL-6, TNFα and/or IL-17 in a subject. By reducing levels of these inflammatory cytokines, the method of the disclosure prevents endothelial dysfunction.

In one example, the STRO-1⁺ cells and/or progeny thereof and/or soluble factors derived therefrom are administered in an amount sufficient to increase levels of angiopoietin I in a subject, e.g., in the endothelium of a subject. As discussed above, levels of angiopoietin II (an antagonist of angiopoietin I) are increased in subjects suffering from endothelial dysfunction. The inventors have shown that STRO-1⁺ MPCs and/or progeny thereof secrete angiopoietin II and reason that this will assist in treating the endothelial dysfunction.

In one example, the STRO-1⁺ cells and/or progeny thereof and/or soluble factors derived therefrom are administered in an amount sufficient to enhance dilation of endothelium (e.g., vascular endothelium) in a subject (e.g., in response to an endothelial dilator such as bradykinin or carbachol).

In one example, the STRO-1⁺ cells and/or progeny thereof and/or soluble factors derived therefrom are administered in an amount sufficient to enhance dilation of endothelium (e.g., vascular endothelium) without enhancing dilation of smooth muscle (e.g., vascular smooth muscle) in a subject (e.g., in response to a smooth muscle dilator such as sodium nitroprusside).

Exemplary dosages of the cells include between 0.1 x 10⁶ to 5 x 10⁶ STRO-1⁺ cells and/or progeny thereof per kilogram. For example, the method comprises administering between 0.3 x 10⁶ to 2 x 10⁶ STRO-1⁺ cells and/or progeny thereof per kilogram.

In one example, the cells are administered at a dose of between about 0.3 x 10⁶ cells/kg to about 4 x 10⁶ cells/kg, such as between about 0.3 x 10⁶ cells/kg to about 2 x 10⁶ cells/kg.

One form of the method involves administering a low dose of STRO-1⁺ cells and/or progeny thereof. Such a low dose is, for example, between 0.1 x 10⁵ to about 0.5 x 10⁶ STRO-1⁺ cells/kg, such as about 0.3 x 10⁶ STRO-1⁺ cells/kg.

In another example, a high dose of cells is administered to the subject. Exemplary dosages include at least about 1.5 x 10⁶ cells/kg. For example, a high dose comprises between about 1.5 x 10⁶ to about 4 x 10⁶ cells/kg. For example, a high dose comprises about 1.5 x 10⁶ or about 2 x 10⁶ cells/kg.

In one example, the cells are administered at a constant body dose, i.e., irrespective of the body weight of the subject.

In one example, the cells are administered at a dose of about 100 million to 300 million cells irrespective of the weight of the subject.

In one example, the cells are administered at a dose of about 100 million to 200 million cells irrespective of the weight of the subject.

In one example, the cells are administered at a dose of about 100 million cells irrespective of the weight of the subject.

In one example, the cells are administered at a dose of about 150 million cells irrespective of the weight of the subject.

In one example, the cells are administered at a dose of about 200 million cells irrespective of the weight of the patient.

In one example, the cells are administered at a dose of about 300 million cells irrespective of the weight of the patient.

In one example, the STRO-1⁺ cells and/or progeny thereof and/or soluble factors derived therefrom are administered in combination with a polysulfated polysaccharide.

In another example, the polysulfated polysaccharide is pentosan polysulfate (PPS) or a pharmaceutically acceptable salt thereof. For example, the PPS is a sodium salt of PPS (NaPPS) or a calcium salt of PPS (CaPPS). For example the salt of PPS is NaPPS.

In one example, the concentration of the PPS is dependent on the number of STRO-1⁺ cells and/or progeny thereof and/or soluble factors derived therefrom, in the composition.

In one example, the concentration of the PPS is about 5ng/ml/million cells-10mg/ml/million cells or 500ng/ml/million cells - 10mg/ml/million cells, 500ng/ml/million cells - 2000µg/ml/million cells, 1µg/ml/million cells - 1000µg/ml/million cells, or 1µg/ml/million cells - 500µg/ml/million cells, 500ng - 10µg/ml/million cells, 1µg - 10µg/ml/million cells, 1µg - 8µg/ml/million cells, 1µg - 6µg/ml/million cells, 1µg - 5µg/ml/million cells, 1µg - 3µg/ml/million cells, 2µg - 6µg/ml/million cells, 2.5µg - 5µg/ml/million cells, or 3µg - 5µg/ml/million cells, 1µg - 100µg/ml/million cells, 1µg - 50µg/ml/million cells, 1µg - 20µg/ml/million cells, 1µg - 15µg/ml/million cells, 10µg - 100µg/ml/million cells, 20µg - 100µg/ml/million cells, or 50µg - 100µg/ml/million cells, 1µg - 1000µg/ml/million cells, 100µg - 800µg/ml/million cells, 100µg - 600µg/ml/million cells, 100µg - 500µg/ml/million cells, 200µg - 500µg/ml/million cells.

In one example, the amount of PPS is between about 1mg-100mg/75 million cells, for example, about 25-75mg/75 million cells, e.g., about 75mg/75 million cells (e.g., 1mg/million cells).

In a further example, the concentration of PPS is about 500ng, 1µg, 2µg, 2.5µg, 5µg, 10µg, 15µg 20µg, 30µg, 40µg, 50µg, 60µg, 70µg, 80µg, 90µg, 100µg, 150µg, 200µg, 250µg, 300µg, 350µg, 400µg, 450µg, 500µg, 550µg, 600µg, 650µg, 700µg, 750µg, 800µg, 850µg, 900µg, 950µg, 1000µg, 1050µg, 1100µg, 1150µg, 1200µg, 1250µg, 1300µg, 1350µg, 1400µg, 1450µg, 1500µg, 1550µg, 1600µg, 1650µg, 1700µg, 1750µg, 1800µg, 1850µg, 1900µg, 1950µg, or 2000µg/ml/million cells.

For example, the total amount of PPS administered is about 1 to 100mg.

For example, the total amount of PPS administered is about 75mg.

In yet a further example, the PPS may be administered in an amount such as to produce a concentration of the PPS of 0.01 to 100 micrograms/ml of composition to be administered to the subject, for example 0.1 to 50 micrograms per ml of composition, 0.1 to 50 micrograms per ml of composition, 0.1 to 10 micrograms per ml of composition, 1 to 10 micrograms per ml of composition, 2 to 8 micrograms per ml of composition, 4 to 6 micrograms per ml of composition, or 4, 5, or 6 micrograms per ml of composition.

In one example, the population enriched for STRO-1⁺ cells and/or progeny thereof and/or soluble factors derived therefrom are administered, with or without PPS, once weekly or less often, such as, once every four weeks or less often.

The present disclosure also contemplates numerous administrations of the cells and/or soluble factors. For example, such a method can involve administering the cells and monitoring the subject to determine when one or more symptoms of endothelial dysfunction occurs or recurs and administering a further dose of the cells and/or soluble factors. Suitable methods for assessing symptoms of endothelial dysfunction will be apparent to the skilled artisan and/or described herein.

In another example, cells and/or soluble factors are administered on a fixed schedule, e.g., once each week or fortnight or three weeks or four weeks or five weeks or six weeks or longer.

In one example, the population enriched for STRO-1⁺ cells and/or progeny cells are autogeneic or allogeneic and/or the soluble factors can be derived from autogeneic or allogeneic cells.

In one example, the population enriched for STRO-1⁺ cells and/or progeny cells have been culture expanded prior to administration and/or prior to obtaining the soluble factors.

In one example, the population enriched for STRO-1⁺ cells are STRO-1^{bright}, and/or express tissue non-specific alkaline phosphatase (TNAP) and/or the progeny cells and/or soluble factors are derived from STRO-1⁺ cells that are STRO-1^{bright} and/or express TNAP.

In one example, the STRO-1⁺ cells and/or progeny cells thereof and/or soluble factors derived therefrom are administered in the form of a composition comprising said STRO-1⁺ cells and/or progeny cells thereof and/or soluble factors derived therefrom and a carrier and/or excipient.

In one example, the subject is a mammal, e.g., a primate, such as a human. In another example, the subject is a non-human animal (e.g., a non-human mammal), such as a domesticated mammal, e.g., a dog or a cat or a horse or a cow or a sheep.

The present disclosure additionally provides a population of cells enriched for STRO-1⁺ cells and/or progeny thereof and/or soluble factors derived therefrom, optionally, in combination with PPS for use in the treatment or prevention of endothelial dysfunction or a condition caused by endothelial dysfunction in a subject.

The present disclosure additionally provides for use of a population of cells enriched for STRO-1⁺ cells and/or progeny thereof and/or soluble factors derived therefrom, optionally, in combination with PPS in the manufacture of a medicament for treating or preventing endothelial dysfunction or a condition caused by endothelial dysfunction in a subject.

### BRIEF DESCRIPTION OF THE DRAWINGS

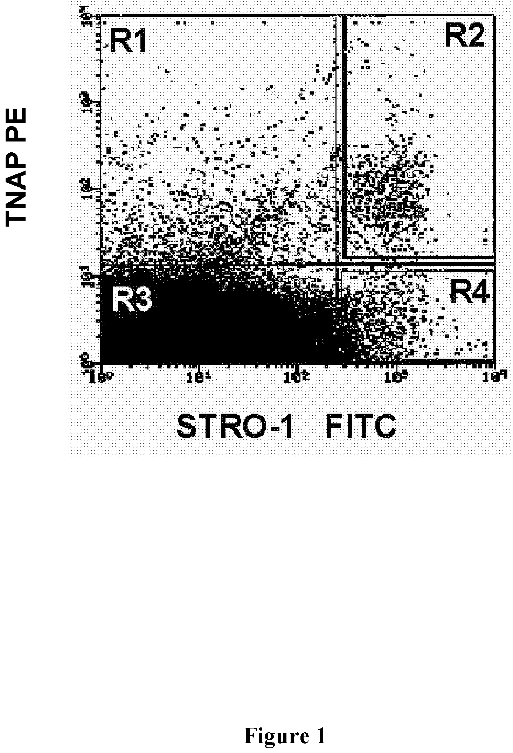

**Figure 1****.** Co-expression of TNAP (STRO-3) and the Mesenchymal Precursor Cell Marker, STRO-1^{bright} by Adult Human bone marrow morphonuclear cells (BMMNC). Dual-color immunofluorescence and flow cytometry was performed by incubation of STRO-1 MACS-selected BMMNC and indirectly labeled with a goat anti-murine IgM antibody coupled to FITC (x axis), and STRO-3 mAb (murine IgG1) indirectly labeled with a goat anti-murine IgG coupled to PE (y axis). The dot plot histogram represents 5 x 10⁴ events collected as listmode data. The vertical and horizontal lines were set to the reactivity levels of <1.0% mean fluorescence obtained with the isotype-matched control antibodies, 1B5 (IgG) and 1A6.12 (IgM) treated under the same conditions. The results demonstrate that a minor population of STRO-1^{bright} cells co-expressed TNAP (upper right quadrant) while the remaining STRO-1⁺ cells failed to react with the STRO-3 mAb.

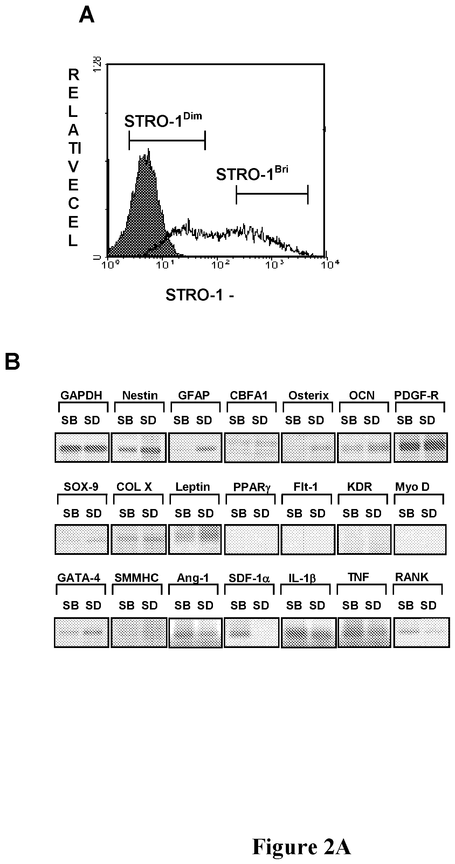

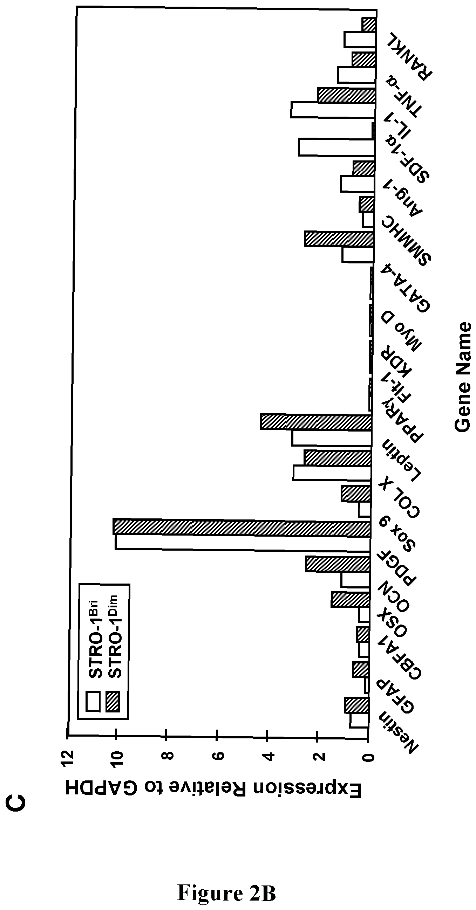

**Figure 2****.** Gene expression profile of STRO-1^{bright} or STRO-1^{dim} progeny of cultured and expanded STRO-1^{bright} MPC. Single cell suspensions of *ex vivo* expanded bone marrow MPC were prepared by trypsin/EDTA treatment. Cells were stained with the STRO-1 antibody which was subsequently revealed by incubation with goat-anti murine IgM-fluorescein isothiocyanate. Total cellular RNA was prepared from purified populations of STRO-1^{dim} or STRO-1^{bright} expressing cells, following fluorescence activated cell sorting (A). Using RNAzolB extraction method, and standard procedures, total RNA was isolated from each subpopulation and used as a template for cDNA synthesis. The expression of various transcripts was assessed by PCR amplification, using a standard protocol as described previously (Gronthos et al. J Cell Sci. 116:1827-1835, 2003). Primers sets used in this study are shown in Table 2. Following amplification, each reaction mixture was analyzed by 1.5% agarose gel electrophoresis, and visualized by ethidium bromide staining (B). Relative gene expression for each cell marker was assessed with reference to the expression of the house-keeping gene, GAPDH, using ImageQant software (C).

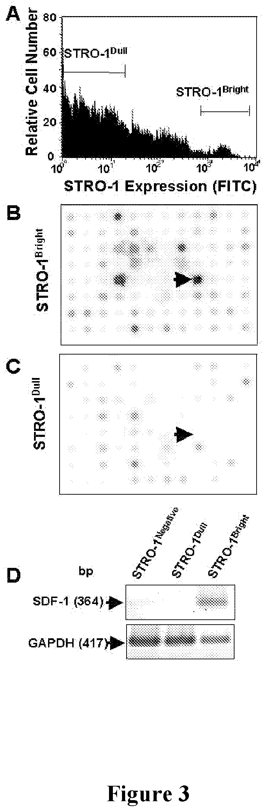

**Figure 3****.** STRO-1^{bright} progeny of cultured and expanded STRO-1⁺ MPC express high levels of SDF-1, STRO-1^{dim} progeny do not. (A) MACS-isolated preparations of STRO-1⁺ BMMNCs were partitioned into different STRO-1 subsets according to the regions, STRO-1^{bright} and STRO-1^{dim/dull} using FACS. Total RNA was prepared from each STRO-1 subpopulation and used to construct a STRO-1^{bright} subtraction hybridization library (B-C). Replicate nitrocellulose filters, which have been blotted with representative PCR products amplified from bacterial clones transformed with STRO-1^{bright} subtracted cDNA. The filters were then probed with either [³²P] deoxycytidine triphosphate (dCTP)-labeled STRO-1^{bright} (B) or STRO-1^{dim/dull} (C) subtracted cDNA. The arrows indicate differential expression of 1 clone containing a cDNA fragment corresponding to human SDF-1. (D) Reverse transcriptase (RT)-PCR analysis demonstrating the relative expression of SDF-1 and glyceraldehyde-3-phosphate dehydrogenase (GAPDH) transcripts in total RNA prepared from freshly MACS/FACS-isolated BMMNC STRO-1 populations prior to culture. bp indicates base pair.

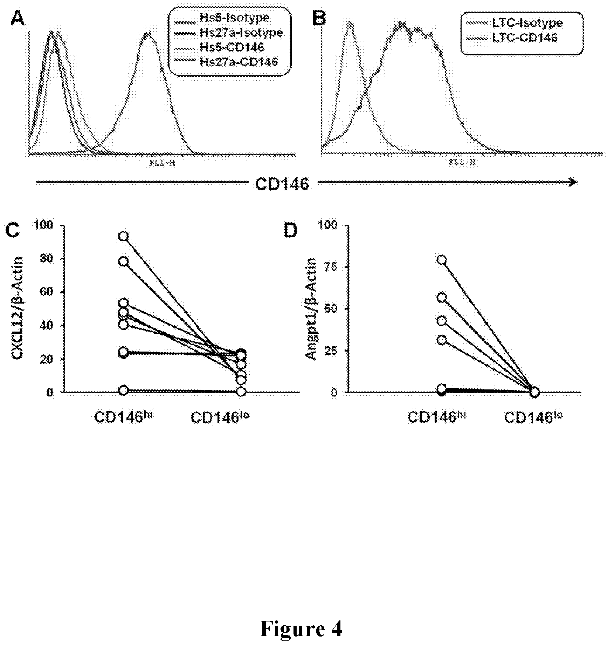

**Figure 4** is a series of graphical representations showing expression of CD146, CXCL12 and Angiopoietin 1 in stromal cells and primary long term cultures. (A) FACS analysis of stromal cell lines HS5 and HS27a for CD146 (MCAM) along with isotype controls. (B) FACS analysis of a primary LTC at first passage for CD146 along with isotype control. The primary LTCs were sorted to CD146^{hi} and CD146^{lo} populations by FACS-aided flow-sorting. Approximate gates for hi and lo populations are also indicated. (C) CXCL12 levels of 9 LTCs sorted to CD146^{hi} and CD146^{lo} and quantitated by RT-PCR (relative to beta-actin, multiplied by a factor of 1000). Paired samples from the same culture are connected by a line. (D) Angiopoietin-1 levels of the same 9 LTCs, also quantitated by RT-PCR (relative to beta-actin, multiplied by a factor of 10000). Experiment in panel A was confirmed in three independent experiments, Experiments in panels B, C and D were confirmed in 9 independent samples. Statistical analysis of the datasets in Panels C and D by Wilcoxon's matched pair test revealed a p-value of <0.01 in both cases.
**Figure 5** shows mean ± SEM for IL-10 levels (ng/ml) in the plasma of treated and untreated sheep over a two week period following induction of endothelial dysfunction by administration of collagen.
**Figure 6** shows mean ± SEM for fibrinogen levels (g/L) in the plasma of treated and untreated sheep over a two week period following induction of endothelial dysfunction by administration of collagen.
**Figure 7** shows mean + SEM for Actin A levels (pg/ml) in the plasma of treated and untreated sheep over a two week period following induction of endothelial dysfunction by administration of collagen.
**Figure 8** shows mean + SEM for C-reactive protein levels (mg/L) in the plasma of treated and untreated sheep over a two week period following induction of endothelial dysfunction by administration of collagen.
**Figure 9** shows the percentage change in C-reactive protein levels in the plasma of treated and untreated sheep over a two week period following induction of endothelial dysfunction by administration of collagen.
**Figure 10** is a series of graphical representations showing percentage relaxation of sheep coronary arteries (A and C) or digital arteries (B and D) following contraction with endothelin-1 and subsequent relaxation with bradykinin (A), carbachol (C) or sodium nitroprusside (B and D) from sheep administered MPCs ("treated") or saline ("controls").
**Figure 11** is a graphical representation showing mean ± SD for peak IL10 levels (ng/mL) relative to baseline in the plasma of MPC ± PPS treated and untreated sheep following induction of endothelial dysfunction by administration of collagen.
**Figure 12** is a graphical representation showing mean ± SEM for the change in fibrinogen levels (g/L) in the plasma, relative to baseline, of MPC ± PPS treated and untreated sheep over a two week period following induction of endothelial dysfunction by administration of collagen.
**Figure 13** is a graphical representation showing mean ± SEM for Actin A levels (pg/ml) in the plasma of MPC ± PPS treated and untreated sheep over a two week period following induction of endothelial dysfunction by administration of collagen.
**Figure 14** is a graphical representation showing the mean ± SEM of the change in C-reactive protein levels in the plasma, relative to baseline, of MPC ± PPS treated and untreated sheep over a two week period following induction of endothelial dysfunction by administration of collagen.
**Figure 15** is a series of graphical representations showing percentage relaxation of sheep coronary arteries (A and C) or digital arteries (B and D) following contraction with endothelin-1 and subsequent relaxation with bradykinin (A), carbachol (C) or sodium nitroprusside (B and D) from sheep administered MPC ± PPS ("treated") or saline ("controls").

### DETAILED DESCRIPTION

### General Techniques and Selected Definitions

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Each embodiment or example described herein is to be applied *mutatis mutandis* to each and every other embodiment unless specifically stated otherwise.

Those skilled in the art will appreciate that the disclosure described herein is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features.

The present disclosure is not to be limited in scope by the specific embodiments described herein, which are intended for the purpose of exemplification only. Functionally-equivalent products, compositions and methods are clearly within the scope of the disclosure, as described herein.

The present disclosure is performed without undue experimentation using, unless otherwise indicated, conventional techniques of molecular biology, microbiology, virology, recombinant DNA technology, peptide synthesis in solution, solid phase peptide synthesis, and immunology. Such procedures are described, for example, in Sambrook, Fritsch & Maniatis, Molecular Cloning: A Laboratory Manual, Cold Spring Harbor Laboratories, New York, Second Edition (1989), whole of Vols I, II, and III; DNA Cloning: A Practical Approach, Vols. I and II (D. N. Glover, ed., 1985), IRL Press, Oxford, whole of text; Oligonucleotide Synthesis: A Practical Approach (M. J. Gait, ed, 1984) IRL Press, Oxford, whole of text, and particularly the papers therein by Gait, ppl-22; Atkinson *et al,* pp35-81; Sproat *et al,* pp 83-115; and Wu et al, pp 135-151; 4. Nucleic Acid Hybridization: A Practical Approach (B. D. Hames & S. J. Higgins, eds., 1985) IRL Press, Oxford, whole of text; Immobilized Cells and Enzymes: A Practical Approach (1986) IRL Press, Oxford, whole of text; Perbal, B., A Practical Guide to Molecular Cloning (1984); Methods In Enzymology (S. Colowick and N. Kaplan, eds., Academic Press, Inc.), whole of series; J.F. Ramalho Ortigao, "The Chemistry of Peptide Synthesis" In: Knowledge database of Access to Virtual Laboratory website (Interactiva, Germany); Sakakibara, D., Teichman, J., Lien, E. Land Fenichel, R.L. (1976). Biochem. Biophys. Res. Commun. 73 336-342; Merrifield, R.B. (1963). J. Am. Chem. Soc. 85, 2149-2154; Barany, G. and Merrifield, R.B. (1979) in The Peptides (Gross, E. and Meienhofer, J. eds.), vol. 2, pp. 1-284, Academic Press, New York. 12. Wünsch, E., ed. (1974) Synthese von Peptiden in Houben-Weyls Metoden der Organischen Chemie (Müler, E., ed.), vol. 15, 4th edn., Parts 1 and 2, Thieme, Stuttgart; Bodanszky, M. (1984) Principles of Peptide Synthesis, Springer-Verlag, Heidelberg; Bodanszky, M. & Bodanszky, A. (1984) The Practice of Peptide Synthesis, Springer-Verlag, Heidelberg; Bodanszky, M. (1985) Int. J. Peptide Protein Res. 25, 449-474; Handbook of Experimental Immunology, VoIs. I-IV (D. M. Weir and C. C. Blackwell, eds., 1986, Blackwell Scientific Publications); and Animal Cell Culture: Practical Approach, Third Edition (John R. W. Masters, ed., 2000), ISBN 0199637970, whole of text.

Throughout this specification, unless the context requires otherwise, the word "comprise", or variations such as "comprises" or "comprising", will be understood to imply the inclusion of a stated step or element or integer or group of steps or elements or integers but not the exclusion of any other step or element or integer or group of elements or integers.

As used herein the term "derived from" shall be taken to indicate that a specified integer may be obtained from a particular source albeit not necessarily directly from that source. In the context of soluble factors derived from stem cells and/or progeny cells thereof, this term shall be taken to mean one or more factors, e.g., proteins, peptides, carbohydrates, etc, produced during *in vitro* culturing of stem cells and/or progeny cells thereof.

As used herein, the term "endothelial dysfunction" will be understood to mean a state (e.g., a systemic state) in a subject which is characterized by an imbalance between vasodilating and vasoconstricting substances and/or which is characterized by a shift in the action of endothelium toward reduced vasodilation and/or a proinflammatory state and/or and prothrombic properties. Methods for detecting endothelial dysfunction will be apparent to the skilled person and/or described herein.

As used herein, the term "condition caused by endothelial dysfunction" will be understood to mean any medical condition in which endothelial dysfunction plays a pathological role. In one example, the condition is a vascular condition. For example, the condition is cardiovascular disease (such as hypertension, coronary artery disease, chronic heart failure and peripheral artery disease), a vascular complication of diabetes (such as nephropathy) or chronic renal failure.

As used herein, the term "nephropathy" will be understood to mean a condition characterized by damage to the kidney and includes non-inflammatory nephropathy (nephrosis) and inflammatory nephropathy (nephritis). Causes of nephropathy include deposition of the IgA antibodies in the glomerulus, administration of analgesics, xanthine oxidase deficiency, and/or long-term exposure to lead or its salts. Chronic conditions that can produce nephropathy include systemic lupus erythematosus (SLE), diabetes mellitus or high blood pressure (hypertension), which lead to diabetic nephropathy and hypertensive nephropathy, respectively.

The term "diabetic nephropathy" (also known as Kimmelstiel-Wilson syndrome, or nodular diabetic glomerulosclerosis and intercapillary glomerulonephritis) will be understood to refer to a progressive kidney condition caused by angiopathy of capillaries in the kidney glomeruli. It is characterized by nephrotic syndrome and diffuse glomerulosclerosis. The first laboratory abnormality generally detected in this condition is a positive microalbuminuria test. Often, the diagnosis is suspected when a routine urinalysis of a person with diabetes shows too much protein in the urine (proteinuria). The urinalysis may also show glucose in the urine, especially if blood glucose is poorly controlled. Serum creatinine may increase as kidney damage progresses. A kidney biopsy can be used to confirm diagnosis, although it is not always necessary if the case is straightforward, with a documented progression of proteinuria over time and presence of diabetic retinopathy on examination of the retina of the eyes.

The term "systemic inflammatory response syndrome" (or "SIRS") is used herein in accordance with its normal meaning, to refer to an inflammatory state of the whole body without a source of infection. There are four major diagnostic symptoms of SIRS, although any two of these are enough for a diagnosis (see e.g. Nystrom (1998) Journal of Antimicrobial Chemotherapy, 41, Suppl A, 1-7).

The term "sepsis" refers to a form of SIRS which is caused by a suspected or proven infection (see e.g. Nystrom (1998) Journal of Antimicrobial Chemotherapy, 41, Suppl. A, 1-7). An infection that leads to sepsis may be caused by e.g. a virus, a fungus, a protozoan or a bacterium.

The term "septic shock" refers to sepsis with hypotension despite adequate resuscitation with fluids (refractory hypotension), along with the presence of perfusion abnormalities (see e.g. Nystrom (1998) Journal of Antimicrobial Chemotherapy, 41, Suppl. A, 1-7).

The term "sepsis-like condition" refers to a state in which a patient presents with symptoms similar to sepsis or septic shock but where the cascade of inflammatory mediators and/or the change in haemodynamic parameters are not primarily or initially caused by an infectious agent. For example, sepsis-like conditions may be seen in a patient with acute or chronic liver failure (see Wasmuth H E, et al. J Hepatol. 2005 February; 42(2): 195-201), patients suffering from post-resuscitation disease after cardiac arrest (see Adrie C et al. Curr Opin Crit Care. 2004 June; 10(3):208-12), patients suffering from sepsis-like symptoms after cancer chemotherapy (see Tsuji E et al. Int J Cancer. 2003 Nov. 1; 107(2):303-8) patients undergoing hyperthermic isolated limb perfusion with recombinant TNF-alpha or similar treatments (see Zwaveling J H et al. Crit Care Med. 1996 May; 24(5):765-70) or sepsis-like illness in neonates (see Griffin M P et al. Pediatr Res. 2003 June; 53(6):920-6.

As used herein, the term "effective amount" shall be taken to mean a sufficient quantity of stem cells and/or progeny cells thereof and/or soluble factors derived therefrom to achieve a significant increase in vascular dilation (e.g., in the presence of an endothelial dilator such as bradykinin or carbachol) in a subject and/or an increase in the level of angiopoietin I in the endothelium in a subject or in a tissue or region thereof a tissue of the subject (e.g., a region of endothelium that is dysfunctioning). This term does not mean that the "effective amount" be tested for these effects each and every time, rather the amount can be established in a preliminary study and based on the results of those studies it can be assumed that the amount will have the same effect in most subjects.

As used herein, the term "therapeutically effective amount" shall be taken to mean a sufficient quantity of stem cells and/or progeny cells thereof and/or soluble factors derived therefrom to treat a condition caused by endothelial dysfunction or a symptom thereof or a clinical sign thereof.

As used herein, the term "prophylactically effective amount" shall be taken to mean a sufficient quantity of stem cells and/or progeny cells thereof and/or soluble factors derived therefrom to prevent or inhibit or delay the onset of a condition caused by endothelial dysfunction or a symptom thereof or a clinical sign thereof.

As used herein, the term "low dose" shall be understood to mean an amount of stem cells and/or progeny thereof less than 1 x 10⁶, yet still sufficient to be an "effective amount" as defined herein and/or a "therapeutically effective amount" and/or a "prophylactically effective amount" as defined herein. For example, a low dose comprises 0.5 x 10⁶ or fewer cells, or 0.4 x 10⁶ or fewer cells or 0.3 x 10⁶ or fewer cells or 0.1 x 10⁶ or fewer cells.

As used herein, the term "high dose" shall be understood to more than 1.5 x 10⁶ cells/kg. For example, a dose comprises between about 1.5 x 10⁶ and about 4 x 10⁶ cells/kg. For example, a high dose comprises about 1.5 x 10⁶ or about 2 x 10⁶/kg.

Reference to a "fixed body dose" will be understood to mean that the stated dose is administered to a subject or population of subjects irrespective of their body weight.

As used herein, the term "treat" or "treatment" or "treating" shall be understood to mean administering a therapeutically effective amount of soluble factors and/or cells and reducing or inhibiting symptom(s) of a condition caused by endothelial dysfunction such that the subject is no longer clinically diagnosed with the condition or such that the level or severity of the condition is reduced.

As used herein, the term "prevent" or "preventing" or "prevention" shall be taken to mean administering a prophylactically effective amount of soluble factors and/or cells and stopping or hindering or delaying the development or progression of a condition caused by endothelial dysfunction.

As used herein, the term "soluble factors" shall be taken to mean any molecule, e.g., protein, peptide, glycoprotein, glycopeptide, lipoprotein, lipopeptide, carbohydrate, etc. produced by stem cells and/or progeny thereof that are water soluble. Such soluble factors may be intracellular and/or secreted by a cell. Such soluble factors may be a complex mixture (e.g., supernatant) and/or a fraction thereof and/or may be a purified factor. In one example of the present disclosure soluble factors are or are contained within supernatant. Accordingly, any example herein directed to administration of one or more soluble factors shall be taken to apply *mutatis mutandis* to the administration of supernatant.

As used herein, the term "supernatant" refers to the non-cellular material produced following the *in vitro* culturing of stem cells and/or progeny thereof in a suitable medium, for example liquid medium. Typically, the supernatant is produced by culturing the cells in the medium under suitable conditions and time, followed by removing the cellular material by a process such as centrifugation. The supernatant may or may not have been subjected to further purification steps before administration. In one example, the supernatant comprises less than 10⁵, for example less than 10⁴, such as less than 10³, e.g., no live cells.

As used herein, the term "normal or healthy individual" shall be taken to mean a subject that does not have endothelial dysfunction as assessed by any method known in the art and/or described herein. In one example, a "normal or healthy individual" does not suffer from any of the symptoms of a condition caused by endothelial dysfunction.

### Endothelial Dysfunction

Numerous tests for assessing endothelial function are known in the art and/or described below and are useful for detecting endothelial dysfunction.

A common parameter assessed when testing endothelial function is endothelium-dependent vasodilation. In coronary arteries, this is performed angiographically by Doppler flow measurements, assessing the effect of endothelium-dependent agonists, mainly acetylcholine (Schachinger et al., Circulation 101: 1899-1906, 2000).

The cold pressor test with measurement of coronary perfusion by positron emission tomography scanning can also be used as a measure of endothelial function (Gokce et al., Am Coll Cardiol 41: 1769-1775, 2003).

Since shear stress is a stimulus to the endothelium to release NO, a noninvasive technique consists of inducing increased shear stress during reactive hyperemia to assess flow-mediated vasodilation of the brachial artery by ultrasound (Celermajer et al., Lancet 340: 1111-1115, 1992).

Since endothelial dysfunction is paralleled by arterial inflammation, markers of endothelial dysfunction include soluble forms of ICAM-1, VCAM-1, and E-selectin, which can be assessed in plasma.

Microalbuminuria has been considered for some time an expression of endothelial dysfunction. Microalbuminuria in most pathologic conditions seems to be a disorder of the capillary wall in the glomerulus with transcapillary escape of albumin. In diabetes, endothelial dysfunction has been correlated with microalbuminuria and may precede its development. Microalbuminuria has also been shown to correlate with markers of endothelial dysfunction.

Quantitative assessment of myocardial blood flow and metabolic activity can be made by positron emission tomography scanning (Gould et al., Circulation 89:1530-1538, 1994). Both basal flow and hyperemic flow (usually to intravenous dipyridamole) can be obtained to calculate coronary flow reserve. Because the increase in myocardial flow is related to adenosine-induced increases and flow-mediated vasodilation, it is in part a measure of endothelial function. This technique is noninvasive and has the advantage of the potential for multiple tests per patient.

Hokanson et al. (IEEE Trans Biomed Eng 22:25-29, 1975) described electrically calibrated plethysmography for direct measurement of limb blood flow. The apparatus is relatively inexpensive and versatile because direct intraarterial infusions of methacholine or acetylcholine assess endothelial function. Because forearm blood flow (ml/min/100 ml) is measured, venous occlusion plethysmography reflects resistance vessel function in the forearm.

### Stem Cells or Progeny Cells, and Supernatant or One or More Soluble Factors Derived Therefrom

As used herein, the term "stem cell" refers to self-renewing cells that are capable of giving rise to phenotypically and genotypically identical daughters as well as at least one other final cell type (e.g., terminally differentiated cells). The term "stem cells" includes totipotential, pluripotential and multipotential cells, as well as progenitor and/or precursor cells derived from the differentiation thereof. The stem cell may be an adult or embryonic stem cell or may be an induced pluripotent stem (iPS).

As used herein, the term "totipotent cell" or "totipotential cell" refers to a cell that is able to form a complete embryo (e.g., a blastocyst).

As used herein, the term "pluripotent cell" or "pluripotential cell" refers to a cell that has complete differentiation versatility, i.e., the capacity to grow into any of the mammalian body's approximately 260 cell types. A pluripotent cell can be self-renewing, and can remain dormant or quiescent within a tissue.

By "multipotential cell" or "multipotent cell" we mean a cell which is capable of giving rise to any of several mature cell types. As used herein, this phrase encompasses adult or embryonic stem cells and progenitor cells, such as mesenchymal precursor cells (MPC) and multipotential progeny of these cells. Unlike a pluripotent cell, a multipotent cell does not have the capacity to form all of the cell types.

As used herein, the term "progenitor cell" refers to a cell that is committed to differentiate into a specific type of cell or to form a specific type of tissue.

As used herein, the phrase "STRO-1⁺ multipotential cells" shall be taken to mean STRO-1⁺ and/or TNAP⁺ progenitor cells capable of forming multipotential cell colonies.

STRO-1⁺ multipotential cells are cells found in bone marrow, blood, dental pulp cells, adipose tissue, skin, spleen, pancreas, brain, kidney, liver, heart, retina, brain, hair follicles, intestine, lung, lymph node, thymus, bone, ligament, tendon, skeletal muscle, dermis, and periosteum; and are capable of differentiating into germ lines such as mesoderm and/or endoderm and/or ectoderm. Thus, STRO-1⁺ multipotential cells are capable of differentiating into a large number of cell types including, but not limited to, adipose, osseous, cartilaginous, elastic, muscular, and fibrous connective tissues. The specific lineage-commitment and differentiation pathway which these cells enter depends upon various influences from mechanical influences and/or endogenous bioactive factors, such as growth factors, cytokines, and/or local microenvironmental conditions established by host tissues. In one embodiment STRO-1⁺ multipotential cells are non-hematopoietic progenitor cells which divide to yield daughter cells that are either stem cells or are precursor cells which in time will irreversibly differentiate to yield a phenotypic cell.

In one example, the STRO-1⁺ cells are enriched from a sample obtained from a subject, e.g., a subject to be treated or a related subject or an unrelated subject (whether of the same species or different). The terms "enriched", "enrichment" or variations thereof are used herein to describe a population of cells in which the proportion of one particular cell type or the proportion of a number of particular cell types is increased when compared with an untreated population of the cells (e.g., cells in their native environment). In one example, a population enriched for STRO-1⁺ cells comprises at least about 0.1% or 0.5% or 1% or 2% or 5% or 10% or 15% or 20% or 25% or 30% or 50% or 75% STRO-1⁺ cells. In this regard, the term "population of cells enriched for STRO-1⁺ cells" will be taken to provide explicit support for the term "population of cells comprising X% STRO-1⁺ cells", wherein X% is a percentage as recited herein. The STRO-1⁺ cells can, in some examples, form clonogenic colonies, e.g. CFU-F (fibroblasts) or a subset thereof (e.g., 50% or 60% or 70% or 70% or 90% or 95%) can have this activity.

In one example, the population of cells is enriched from a cell preparation comprising STRO-1⁺ cells in a selectable form. In this regard, the term "selectable form" will be understood to mean that the cells express a marker (e.g., a cell surface marker) permitting selection of the STRO-1⁺ cells. The marker can be STRO-1, but need not be. For example, as described and/or exemplified herein, cells (e.g., MPCs) expressing STRO-2 and/or STRO-3 (TNAP) and/or STRO-4 and/or VCAM-1 and/or CD146 and/or 3G5 also express STRO-1 (and can be STRO-1^{bright}). Accordingly, an indication that cells are STRO-1⁺ does not mean that the cells are selected by STRO-1 expression. In one example, the cells are selected based on at least STRO-3 expression, e.g., they are STRO-3⁺ (TNAP⁺).

Reference to selection of a cell or population thereof does not necessarily require selection from a specific tissue source. As described herein STRO-1⁺ cells can be selected from or isolated from or enriched from a large variety of sources. That said, in some examples, these terms provide support for selection from any tissue comprising STRO-1⁺ cells (e.g., MPCs) or vascularized tissue or tissue comprising pericytes (e.g., STRO-1⁺ pericytes) or any one or more of the tissues recited herein.

In one example, the cells used in the present disclosure express one or more markers individually or collectively selected from the group consisting of TNAP⁺, VCAM-1⁺, THY-1⁺, STRO-2⁺, STRO-4⁺ (HSP-94β), CD45⁺, CD146⁺, 3G5⁺ or any combination thereof.

By "individually" is meant that the disclosure encompasses the recited markers or groups of markers separately, and that, notwithstanding that individual markers or groups of markers may not be separately listed herein the accompanying claims may define such marker or groups of markers separately and divisibly from each other.

By "collectively" is meant that the disclosure encompasses any number or combination of the recited markers or groups of peptides, and that, notwithstanding that such numbers or combinations of markers or groups of markers may not be specifically listed herein the accompanying claims may define such combinations or subcombinations separately and divisibly from any other combination of markers or groups of markers.

For example, the STRO-1⁺ cells are STRO-1^{bright} (*syn.* STRO-1^{bri}). In one example, the Stro-1^{bri} cells are preferentially enriched relative to STRO-1^{dim} or STRO-1^{intermediate} cells.

For example, the STRO-1^{bright} cells are additionally one or more of TNAP⁺, VCAM-1⁺, THY-1⁺, STRO-2⁺, STRO-4⁺ (HSP-90β) a ⁺. For example, the cells are selected for one or more of the foregoing markers and/or shown to express one or more of the foregoing markers. In this regard, a cell shown to express a marker need not be specifically tested, rather previously enriched or isolated cells can be tested and subsequently used, isolated or enriched cells can be reasonably assumed to also express the same marker.

In one example, the mesenchymal precursor cells are perivascular mesenchymal precursor cells as defined in WO 2004/85630. For example, the mesenchymal precursor cells express a marker of a perivascular cell, e.g., the cells are STRO-1⁺ or STRO-1^{bright} and/or 3G5⁺. In one example, the cells are or were previously or are progeny of cells that were isolated from vascularized tissue or organs or parts thereof.

A cell that is referred to as being "positive" for a given marker it may express either a low (lo or dim) or a high (bright, bri) level of that marker depending on the degree to which the marker is present on the cell surface, where the terms relate to intensity of fluorescence or other marker used in the sorting process of the cells. The distinction of lo (or dim or dull) and bri will be understood in the context of the marker used on a particular cell population being sorted. A cell that is referred to as being "negative" for a given marker is not necessarily completely absent from that cell. This term means that the marker is expressed at a relatively very low level by that cell, and that it generates a very low signal when detectably labeled or is undetectable above background levels, e.g., levels detected using an isotype control antibody.

The term "bright", when used herein, refers to a marker on a cell surface that generates a relatively high signal when detectably labeled. Whilst not wishing to be limited by theory, it is proposed that "bright" cells express more of the target marker protein (for example the antigen recognized by STRO-1) than other cells in the sample. For instance, STRO-1^{bri} cells produce a greater fluorescent signal, when labeled with a FITC-conjugated STRO-1 antibody as determined by fluorescence activated cell sorting (FACS) analysis, than non-bright cells (STRO-1^{dull/dim}). In one example, "bright" cells constitute at least about 0.1% of the most brightly labeled bone marrow mononuclear cells contained in the starting sample. In other examples, "bright" cells constitute at least about 0.1%, at least about 0.5%, at least about 1%, at least about 1.5%, or at least about 2%, of the most brightly labeled bone marrow mononuclear cells contained in the starting sample. In an example, STRO-1^{bright} cells have 2 log magnitude higher expression of STRO-1 surface expression relative to "background", namely cells that are STRO-1⁻. By comparison, STRO-1^{dim} and/or STRO-1^{intermediate} cells have less than 2 log magnitude higher expression of STRO-1 surface expression, typically about 1 log or less than "background".

As used herein the term "TNAP" is intended to encompass all isoforms of tissue non-specific alkaline phosphatase. For example, the term encompasses the liver isoform (LAP), the bone isoform (BAP) and the kidney isoform (KAP). In one example, the TNAP is BAP. In one example, TNAP as used herein refers to a molecule which can bind the STRO-3 antibody produced by the hybridoma cell line deposited with ATCC on 19 December 2005 under the provisions of the Budapest Treaty under deposit accession number PTA-7282.

Furthermore, in one example, the STRO-1⁺ cells are capable of giving rise to clonogenic CFU-F.

In one example, a significant proportion of the STRO-1⁺ multipotential cells are capable of differentiation into at least two different germ lines. Non-limiting examples of the lineages to which the multipotential cells may be committed include bone precursor cells; hepatocyte progenitors, which are multipotent for bile duct epithelial cells and hepatocytes; neural restricted cells, which can generate glial cell precursors that progress to oligodendrocytes and astrocytes; neuronal precursors that progress to neurons; precursors for cardiac muscle and cardiomyocytes, glucose-responsive insulin secreting pancreatic beta cell lines. Other lineages include, but are not limited to, odontoblasts, dentin-producing cells and chondrocytes, and precursor cells of the following: retinal pigment epithelial cells, fibroblasts, skin cells such as keratinocytes, dendritic cells, hair follicle cells, renal duct epithelial cells, smooth and skeletal muscle cells, testicular progenitors, vascular endothelial cells, tendon, ligament, cartilage, adipocyte, fibroblast, marrow stroma, cardiac muscle, smooth muscle, skeletal muscle, pericyte, vascular, epithelial, glial, neuronal, astrocyte and oligodendrocyte cells.

In another example, the STRO-1⁺ cells are not capable of giving rise, upon culturing, to hematopoietic cells.

In one example, the cells are taken from the subject to be treated, cultured *in vitro* using standard techniques and used to obtain supernatant or soluble factors or expanded cells for administration to the subject as an autologous or allogeneic composition. In an alternative example, cells of one or more of the established human cell lines are used. In another useful example of the disclosure, cells of a non-human animal (or if the patient is not a human, from another species) are used.

The present disclosure also contemplates use of supernatant or soluble factors obtained or derived from STRO-1⁺ cells and/or progeny cells thereof (the latter also being referred to as expanded cells) which are produced from *in vitro* culture. Expanded cells of the disclosure may have a wide variety of phenotypes depending on the culture conditions (including the number and/or type of stimulatory factors in the culture medium), the number of passages and the like. In certain examples, the progeny cells are obtained after about 2, about 3, about 4, about 5, about 6, about 7, about 8, about 9, or about 10 passages from the parental population. However, the progeny cells may be obtained after any number of passages from the parental population.

The progeny cells may be obtained by culturing in any suitable medium. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase that cells growing on a petri dish or other solid or semisolid support are exposed to. The term "medium" also refers to material that is intended for use in a cell culture, even if it has not yet been contacted with cells. In other words, a nutrient rich liquid prepared for bacterial culture is a medium. A powder mixture that when mixed with water or other liquid becomes suitable for cell culture may be termed a "powdered medium".

In an example, progeny cells useful for the methods of the disclosure are obtained by isolating TNAP⁺ STRO-1⁺ cells from bone marrow using magnetic beads labeled with the STRO-3 antibody, and then culture expanding the isolated cells (see Gronthos et al. Blood 85: 929-940, 1995 for an example of suitable culturing conditions).

In one example, such expanded cells (progeny) (for example, after at least 5 passages) can be TNAP⁻, CC9⁺, HLA class I⁺, HLA class II⁻, CD14⁻, CD19⁻, CD3⁻, CD11a⁻c⁻, CD31⁻, CD86⁻, CD34⁻ and/or CD80⁻. However, it is possible that under different culturing conditions to those described herein that the expression of different markers may vary. Also, whilst cells of these phenotypes may predominate in the expended cell population it does not mean that there is a minor proportion of the cells that do not have this phenotype(s) (for example, a small percentage of the expanded cells may be CC9⁻). In one example, expanded cells still have the capacity to differentiate into different cell types.

In one example, an expended cell population used to obtain supernatant or soluble factors, or cells *per se,* comprises cells wherein at least 25%, for example at least 50%, of the cells are CC9⁺.

In another example, an expanded cell population used to obtain supernatant or soluble factors, or cells *per se,* comprises cells wherein at least 40%, for example at least 45%, of the cells are STRO-1⁺.

In a further example, the expanded cells may express one or more markers collectively or individually selected from the group consisting of LFA-3, THY-1, VCAM-1, ICAM-1, PECAM-1, P-selectin, L-selectin, 3G5, CD49a/CD49b/CD29, CD49c/CD29, CD49d/CD29, CD 90, CD29, CD18, CD61, integrin beta 6-19, thrombomodulin, CD10, CD13, SCF, PDGF-R, EGF-R, IGF1-R, NGF-R, FGF-R, Leptin-R (STRO-2 = Leptin-R), RANKL, STRO-4 (HSP-90β), STRO-1^{bright} and CD146 or any combination of these markers.

In one example, the progeny cells are Multipotential Expanded STRO-1⁺ Multipotential cells Progeny (MEMPs) as defined and/or described in WO 2006/032092. Methods for preparing enriched populations of STRO-1⁺ multipotential cells from which progeny may be derived are described in WO 01/04268 and WO 2004/085630. In an *in vitro* context STRO-1⁺ multipotential cells will rarely be present as an absolutely pure preparation and will generally be present with other cells that are tissue specific committed cells (TSCCs). WO 01/04268 refers to harvesting such cells from bone marrow at purity levels of about 0.1% to 90%. The population comprising MPCs from which progeny are derived may be directly harvested from a tissue source, or alternatively it may be a population that has already been expanded *ex vivo.*

For example, the progeny may be obtained from a harvested, unexpanded, population of substantially purified STRO-1⁺ multipotential cells, comprising at least about 0.1, 1, 5, 10, 20, 30, 40, 50, 60, 70, 80 or 95% of total cells of the population in which they are present. This level may be achieved, for example, by selecting for cells that are positive for at least one marker individually or collectively selected from the group consisting of TNAP, STRO-4 (HSP-90β), STRO-1^{bright}, 3G5⁺, VCAM-1, THY-1, CD146 and STRO-2.

MEMPS can be distinguished from freshly harvested STRO-1⁺ multipotential cells in that they are positive for the marker STRO-1^{bri} and negative for the marker Alkaline phosphatase (ALP). In contrast, freshly isolated STRO-1⁺ multipotential cells are positive for both STRO-1^{bri} and ALP. In one example of the present disclosure, at least 15%, 20%, 30%, 40%, 50%, 60%, 70%, 80%, 90% or 95% of the administered cells have the phenotype STRO-1^{bri}, ALP⁻. In a further example the MEMPS are positive for one or more of the markers Ki67, CD44 and/or CD49c/CD29, VLA-3, α3β1. In yet a further example the MEMPs do not exhibit TERT activity and/or are negative for the marker CD18.

The STRO-1⁺ cell starting population may be derived from any one or more tissue types set out in WO 01/04268 or WO 2004/085630, namely bone marrow, dental pulp cells, adipose tissue and skin, or perhaps more broadly from adipose tissue, teeth, dental pulp, skin, liver, kidney, heart, retina, brain, hair follicles, intestine, lung, spleen, lymph node, thymus, pancreas, bone, ligament, bone marrow, tendon and skeletal muscle.

It will be understood that in performing methods described in the present disclosure, separation of cells carrying any given cell surface marker can be effected by a number of different methods, however, some exemplary methods rely upon binding a binding agent (e.g., an antibody or antigen binding fragment thereof) to the marker concerned followed by a separation of those that exhibit binding, being either high level binding, or low level binding or no binding. The most convenient binding agents are antibodies or antibody-based molecules, for example monoclonal antibodies or based on monoclonal antibodies (e.g., proteins comprising antigen binding fragments thereof) because of the specificity of these latter agents. Antibodies can be used for both steps, however other agents might also be used, thus ligands for these markers may also be employed to enrich for cells carrying them, or lacking them.

The antibodies or ligands may be attached to a solid support to allow for a crude separation. In some examples the separation techniques maximize the retention of viability of the fraction to be collected. Various techniques of different efficacy may be employed to obtain relatively crude separations. The particular technique employed will depend upon efficiency of separation, associated cytotoxicity, ease and speed of performance, and necessity for sophisticated equipment and/or technical skill. Procedures for separation may include, but are not limited to, magnetic separation, using antibody-coated magnetic beads, affinity chromatography and "panning" with antibody attached to a solid matrix. Techniques providing accurate separation include but are not limited to FACS. Methods for performing FACS will be apparent to the skilled artisan.

Antibodies against each of the markers described herein are commercially available (e.g., monoclonal antibodies against STRO-1 are commercially available from R&D Systems, USA), available from ATCC or other depositary organization and/or can be produced using art recognized techniques.

In one example, the method for isolating STRO-1⁺ cells comprises a first step being a solid phase sorting step utilizing for example magnetic activated cell sorting (MACS) recognizing high level expression of STRO-1. A second sorting step can then follow, should that be desired, to result in a higher level of precursor cell expression as described in patent specification WO 01/14268. This second sorting step might involve the use of two or more markers.

The method obtaining STRO-1⁺ cells might also include the harvesting of a source of the cells before the first enrichment step using known techniques. Thus the tissue will be surgically removed. Cells comprising the source tissue will then be separated into a so called single cells suspension. This separation may be achieved by physical and or enzymatic means.

Once a suitable STRO-1⁺ cell population has been obtained, it may be cultured or expanded by any suitable means to obtain MEMPs.

In one example, the cells are taken from the subject to be treated, cultured *in vitro* using standard techniques and used to obtain supernatant or soluble factors or expanded cells for administration to the subject as an autologous or allogeneic composition. In an alternative example, cells of one or more of the established human cell lines are used to obtain the supernatant or soluble factors. In another useful example of the disclosure, cells of a non-human animal (or if the patient is not a human, from another species) are used to obtain supernatant or soluble factors.

Methods and uses of the present disclosure can be practiced using cells from any non-human animal species, including but not limited to non-human primate cells, ungulate, canine, feline, lagomorph, rodent, avian, and fish cells. Primate cells with which the disclosure may be performed include but are not limited to cells of chimpanzees, baboons, cynomolgus monkeys, and any other New or Old World monkeys. Ungulate cells with which the disclosure may be performed include but are not limited to cells of bovines, porcines, ovines, caprines, equines, buffalo and bison. Rodent cells with which the disclosure may be performed include but are not limited to mouse, rat, guinea pig, hamster and gerbil cells. Examples of lagomorph species with which the disclosure may be performed include domesticated rabbits, jack rabbits, hares, cottontails, snowshoe rabbits, and pikas. Chickens (*Gallus gallus*) are an example of an avian species with which the disclosure may be performed.

In one example, the cells are human cells.

Cells useful for the methods of the disclosure may be stored before use, or before obtaining the supernatant or soluble factors. Methods and protocols for preserving and storing of eukaryotic cells, and in particular mammalian cells, are known in the art (cf., for example, Pollard, J. W. and Walker, J. M. (1997) Basic Cell Culture Protocols, Second Edition, Humana Press, Totowa, N.J.; Freshney, R. I. (2000) Culture of Animal Cells, Fourth Edition, Wiley-Liss, Hoboken, N.J.). Any method maintaining the biological activity of the isolated stem cells such as mesenchymal stem/progenitor cells, or progeny thereof, may be utilized in connection with the present disclosure. In one example, the cells are maintained and stored by using cryo-preservation.

### Polysulfated polysaccharides

Polysulfated polysaccharides may be any naturally occurring or semi-synthetic/synthetic polysulfated polysaccharide or a biologically active fragment thereof that contains two or more sugar rings or carbohydrate structures to which one or more sulfate ester groups are covalently attached as exemplified by heparin and pentosan polysulfate.

In one example, the STRO-1⁺ cells and/or progeny thereof and/or soluble factors derived therefrom are administered in combination with a polysulfated polysaccharide.

In another example, the polysulfated polysaccharide is PPS and pharmaceutically acceptable salts thereof.

In one example, the PPS is isolated from beechwood hemicellulose (*Fagus silvatica*) with a linear xylan (pentosan) backbone of pentosan polysulfate contains on average one 4-O-methyl-glucuronate side chain linked to the 2-position on every tenth xylose (pentose) ring. For example, the pentosan polysulfate and pharmaceutically acceptable salts thereof has the following structure:

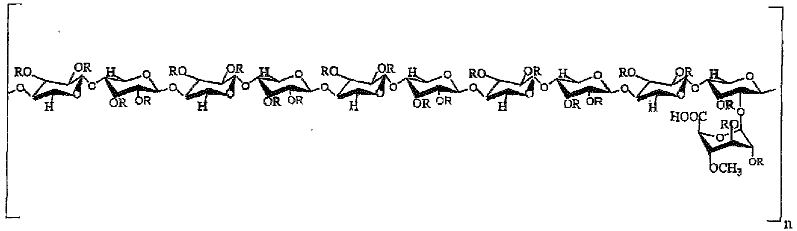

In one example the particular complexing ions (to produce the salt of PPS) may be selected from the group consisting of the alkali metals, e. g. Na⁺, and K+, alkaline earth metals, e. g.Ca²⁺, Zn²⁺, Mg²⁺, Ba²⁺, as well as Ag⁺, Au⁺, Pb²⁺, Cu²⁺, Au²⁺, Pd²⁺, Pd⁴⁺, Pt⁴⁺, Pt²⁺, trivalent metal ions, and quaternary ammonium compound complexes. Examples of the latter compounds are pyridinium chloride, tetraalkyl ammonium chloride, choline chloride, cetylpyridinium chloride, N-cetyl-N, N, N-trialkylammonium chloride or their derivatives.

For example, the complexing ion is sodium, i.e., the PPS is NaPPS. For example the NaPPS is SP54, manufactured by Bene Pharmachem, Germany.

In one example, the PPS improves the viability of progenitor cells, enhance the cryopreservation of progenitor cells, regulate the proliferation of progenitor cells and/or regulate the differentiation of progenitor cells.

### Genetically-Modified Cells

In one example, the stem cells and/or progeny cells thereof are genetically modified, e.g., to express and/or secrete a protein of interest. For example, the cells are engineered to express a protein useful in the treatment of endothelial dysfunction, e.g., Angiopoietin I or nitric oxide synthase (e.g., eNOS or iNOS).

Methods for genetically modifying a cell will be apparent to the skilled artisan. For example, a nucleic acid that is to be expressed in a cell is operably-linked to a promoter for inducing expression in the cell. For example, the nucleic acid is linked to a promoter operable in a variety of cells of a subject, such as, for example, a viral promoter, e.g., a CMV promoter (e.g., a CMV-IE promoter) or a SV-40 promoter. Additional suitable promoters are known in the art and shall be taken to apply *mutatis mutandis* to the present example of the disclosure.

In one example, the nucleic acid is provided in the form of an expression construct. As used herein, the term "expression construct" refers to a nucleic acid that has the ability to confer expression on a nucleic acid (e.g. a reporter gene and/or a counter-selectable reporter gene) to which it is operably connected, in a cell. Within the context of the present disclosure, it is to be understood that an expression construct may comprise or be a plasmid, bacteriophage, phagemid, cosmid, virus sub-genomic or genomic fragment, or other nucleic acid capable of maintaining and/or replicating heterologous DNA in an expressible format.

Methods for the construction of a suitable expression construct for performance of the disclosure will be apparent to the skilled artisan and are described, for example, in Ausubel et al (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001). For example, each of the components of the expression construct is amplified from a suitable template nucleic acid using, for example, PCR and subsequently cloned into a suitable expression construct, such as for example, a plasmid or a phagemid.

Vectors suitable for such an expression construct are known in the art and/or described herein. For example, an expression vector suitable for the method of the present disclosure in a mammalian cell is, for example, a vector of the pcDNA vector suite supplied by Invitrogen, a vector of the pCI vector suite (Promega), a vector of the pCMV vector suite (Clontech), a pM vector (Clontech), a pSI vector (Promega), a VP 16 vector (Clontech) or a vector of the pcDNA vector suite (Invitrogen).

The skilled artisan will be aware of additional vectors and sources of such vectors, such as, for example, Life Technologies Corporation, Clontech or Promega.

Means for introducing the isolated nucleic acid molecule or a gene construct comprising same into a cell for expression are known to those skilled in the art. The technique used for a given organism depends on the known successful techniques. Means for introducing recombinant DNA into cells include microinjection, transfection mediated by DEAE-dextran, transfection mediated by liposomes such as by using lipofectamine (Gibco, MD, USA) and/or cellfectin (Gibco, MD, USA), PEG-mediated DNA uptake, electroporation and microparticle bombardment such as by using DNA-coated tungsten or gold particles (Agracetus Inc., WI, USA) amongst others.

Alternatively, an expression construct of the disclosure is a viral vector. Suitable viral vectors are known in the art and commercially available. Conventional viral-based systems for the delivery of a nucleic acid and integration of that nucleic acid into a host cell genome include, for example, a retroviral vector, a lentiviral vector or an adeno-associated viral vector. Alternatively, an adenoviral vector is useful for introducing a nucleic acid that remains episomal into a host cell. Viral vectors are an efficient and versatile method of gene transfer in target cells and tissues. Additionally, high transduction efficiencies have been observed in many different cell types and target tissues.

For example, a retroviral vector generally comprises cis-acting long terminal repeats (LTRs) with packaging capacity for up to 6-10 kb of foreign sequence. The minimum cis-acting LTRs are sufficient for replication and packaging of a vector, which is then used to integrate the expression construct into the target cell to provide long term expression. Widely used retroviral vectors include those based upon murine leukemia virus (MuLV), gibbon ape leukemia virus (GaLV), simian immunodeficiency virus (SrV), human immunodeficiency virus (HIV), and combinations thereof (see, e.g., Buchscher et al., J Virol. 56:2731-2739 (1992); Johann et al, J. Virol. 65:1635-1640 (1992); Sommerfelt et al, Virol. 76:58-59 (1990); Wilson et al, J. Virol. 63:274-2318 (1989); Miller et al., J. Virol. 65:2220-2224 (1991); PCT/US94/05700; Miller and Rosman BioTechniques 7:980-990, 1989; Miller, A. D. Human Gene Therapy 7:5-14, 1990; Scarpa et al Virology 75:849-852, 1991; Burns et al. Proc. Natl. Acad. Sci USA 90:8033-8037, 1993).

Various adeno-associated virus (AAV) vector systems have also been developed for nucleic acid delivery. AAV vectors can be readily constructed using techniques known in the art. See, e.g., U.S. Pat. Nos. 5,173,414 and 5,139,941; International Publication Nos. WO 92/01070 and WO 93/03769; Lebkowski et al. Molec. Cell. Biol. 5:3988-3996, 1988; Vincent et al. (1990) Vaccines 90 (Cold Spring Harbor Laboratory Press);Carter Current Opinion in Biotechnology 5:533-539, 1992; Muzyczka. Current Topics in Microbiol, and Immunol. 158:91-129, 1992; Kotin, Human Gene Therapy 5:793-801, 1994; Shelling and Smith Gene Therapy 7:165-169, 1994; and Zhou et al. J Exp. Med. 179:1867-1875, 1994.

Additional viral vectors useful for delivering an expression construct of the disclosure include, for example, those derived from the pox family of viruses, such as vaccinia virus and avian poxvirus or an alphavirus or a conjugate virus vector (e.g. that described in Fisher-Hoch et al., Proc. Natl Acad. Sci. USA 56:317-321, 1989).

### Assaying Therapeutic/Prophylactic Potential of Cells and Soluble Factors

Various models for studying endothelial function/dysfunction are known in the art. Exemplary *in vitro* models include:
- Culturing endothelial cells in high glucose medium, which results in suppression of proliferation, increased expression of adhesion molecules and increased apoptosis; and
- An organ culture of mesenteric artery, e.g., as described in Alm et al., BMC Cardiovascular Disorders, 2: 8, 2002

*In vivo* models of endothelial function/dysfunction include:
- A mouse model of hyperhomocystinemia as described in Eberhardt et al., J Clin Invest 106: 483-491, 2000;
- A rat hypertension model produced using goldblatt techniques such as 2-kidney 1-clip model and 1-kidney 1-clip model, which have been demonstrated to increase arterial blood pressure, total peripheral resistance (TPR) and decrease endothelium dependent relaxation to acetylcholine (Ach) (Share et al., Clin. Exp. Hypertens., 4: 1261-1270, 2982; Sventek et al., Hypertensive, 27: 49-55, 1996).
- Uninephrectomy in rodents followed by administration of DOCA salt (40 mg kg-1, s.c.) in olive oil along with 1% NaCl and 0.5% KCl twice weekly for 6 weeks has produced vascular endothelial dysfunction (Shah and Singh, Naun. Schmie. Arch. Pharmacol., 373: 221-229, 2006).
- Treatment of rodents with L-NAME (an eNOS inhibitor) (50 mg kg-1 day-1) for 6 weeks has been shown to increase blood pressure and reduce endothelium dependent relaxation in rats (Kung et al., Hypertensive, 26: 744-751, 1995).
- Infusion of angiotensin-II (0.7 mg kg-1 day-1) into rodents for 5 days has been noted to increase systolic blood pressure, generation of superoxide anion and cause impairment of Ach-induced relaxation (Rajagopalan et al., J. Clin. Invest., 97: 1916-1923, 1996).
- Chronic administration of ethinyl estradiol (1.5 mg kg-1 day-1) to rodents has been shown to increase blood pressure and consequently reduce endothelium dependent relaxation (Thakre et al., Ind. J. Pharmacol., 32: 15-20, 2000).
- Rodents fed a moderately high fat diet administration for 10 weeks have been shown to develop vascular endothelial dysfunction characterized by hypertension, increase in reactive oxygen species (ROS) and lipid peroxidation (Dobrian et al., Hypertensive, 37: 554-560, 2001).
- Administration of streptozotocin (55 mg kg-1, i.p. once) in rats produced diabetes and consequently induced vascular endothelial dysfunction (Shah and Singh, Mol. Cell. Biochem., 283: 191-199, 2006).

Additional models of endothelial dysfunction are described, for example, in Balakumar et al., Trends in Medical Research, 2: 12-20, 2007.

### Cellular Compositions

In one example of the present disclosure STRO-1⁺ cells and/or progeny cells thereof are administered in the form of a composition. For example, such a composition comprises a pharmaceutically acceptable carrier and/or excipient.

The terms "carrier" and "excipient" refer to compositions of matter that are conventionally used in the art to facilitate the storage, administration, and/or the biological activity of an active compound (see, e.g., Remington's Pharmaceutical Sciences, 16th Ed., Mac Publishing Company (1980)). A carrier may also reduce any undesirable side effects of the active compound. A suitable carrier is, for example, stable, e.g., incapable of reacting with other ingredients in the carrier. In one example, the carrier does not produce significant local or systemic adverse effects in recipients at the dosages and concentrations employed for treatment.

Suitable carriers for the present disclosure include those conventionally used, e.g., water, saline, aqueous dextrose, lactose, Ringer's solution, a buffered solution, hyaluronan and glycols are exemplary liquid carriers, particularly (when isotonic) for solutions. Suitable pharmaceutical carriers and excipients include starch, cellulose, glucose, lactose, sucrose, gelatin, malt, rice, flour, chalk, silica gel, magnesium stearate, sodium stearate, glycerol monostearate, sodium chloride, glycerol, propylene glycol, water, ethanol, and the like.

In another example, a carrier is a media composition, e.g., in which a cell is grown or suspended. For example, such a media composition does not induce any adverse effects in a subject to whom it is administered.

Exemplary carriers and excipients do not adversely affect the viability of a cell and/or the ability of a cell to reduce, prevent or delay inflammatory joint disease.

In one example, the carrier or excipient provides a buffering activity to maintain the cells and/or soluble factors at a suitable pH to thereby exert a biological activity, e.g., the carrier or excipient is phosphate buffered saline (PBS). PBS represents an attractive carrier or excipient because it interacts with cells and factors minimally and permits rapid release of the cells and factors, in such a case, the composition of the disclosure may be produced as a liquid for direct application to the blood stream or into a tissue or a region surrounding or adjacent to a tissue, e.g., by injection.

STRO-1⁺ cells and/or progeny cells thereof can also be incorporated or embedded within scaffolds that are recipient-compatible and which degrade into products that are not harmful to the recipient. These scaffolds provide support and protection for cells that are to be transplanted into the recipient subjects. Natural and/or synthetic biodegradable scaffolds are examples of such scaffolds.

A variety of different scaffolds may be used successfully in the practice of the disclosure. Exemplary scaffolds include, but are not limited to biological, degradable scaffolds. Natural biodegradable scaffolds include collagen, fibronectin, and laminin scaffolds. Suitable synthetic material for a cell transplantation scaffold should be able to support extensive cell growth and cell function. Such scaffolds may also be resorbable. Suitable scaffolds include polyglycolic acid scaffolds, e.g., as described by Vacanti, et al. J. Ped. Surg. 23:3-9 1988; Cima, et al. Biotechnol. Bioeng. 38:145 1991; Vacanti, et al. Plast. Reconstr. Surg. 88:753-9 1991; or synthetic polymers such as polyanhydrides, polyorthoesters, and polylactic acid.

In another example, the cells may be administered in a gel scaffold (such as Gelfoam from Upjohn Company.

The cellular compositions useful for methods described herein may be administered alone or as admixtures with other cells. Cells that may be administered in conjunction with the compositions of the present disclosure include, but are not limited to, other multipotent or pluripotent cells or stem cells, or bone marrow cells. The cells of different types may be admixed with a composition of the disclosure immediately or shortly prior to administration, or they may be co-cultured together for a period of time prior to administration.

In one example, the composition comprises an effective amount or a therapeutically or prophylactically effective amount of cells. For example, the composition comprises about 1 x 10⁵ STRO-1⁺ cells/kg to about 1 x 10⁷ STRO-1⁺ cells/kg or about 1 x 10⁶ STRO-1⁺ cells/kg to about 5 x 10⁶ STRO-1⁺ cells/kg. The exact amount of cells to be administered is dependent upon a variety of factors, including the age, weight, and sex of the patient, and the extent and severity of the inflammatory joint disease

In one example, a low dose of cells is administered to the subject. Exemplary dosages include between about 0.1 x 10⁴ to about 0.5 x 10⁶ cells per kg, for example, between about 0.1 x 10⁵ to about 0.5 x 10⁶ cells per kg, such as, between about 0.5 x 10⁵ to about 0.5 x 10⁶ cells per kg, for example, between about 0.1 x 10⁶ to about 0.5 x 10⁶ cells per kg, e.g., about 0.2 x 10⁶ or 0.3 x 10⁶ or 0.4 x 10⁶ cells per kg.

In one example, a high dose of cells is administered to the subject. Exemplary dosages include at least about 1.5 x 10⁶ cells/kg. For example, a high dose comprises between about 1.5 x 10⁶ to about 6 x 10⁶ cells/kg, such as between about 1.5 x 10⁶ to about 5 x 10⁶ cells/kg, for example, between about 1.5 x 10⁶ to about 4 x 10⁶ cells/kg, for example, between about 1.5 x 10⁶ to about 3 x 10⁶ cells/kg. For example, a high dose comprises about 1.5 x 10⁶ or about 2 x 10⁶ cells/kg.

In one example, the cells are administered as a total cell number dose irrespective of the patient's weight.

For example, in one example, the cells are administered at a dose of between about 100 million to 300 million cells irrespective of the weight of the patient.

For example, in one example, the cells are administered at a dose of between about 100 million to 200 million cells irrespective of the weight of the patient.

In one example, the cells are administered at a dose of about 100 million cells irrespective of the weight of the patient.

In one example, the cells are administered at a dose of about 150 million cells irrespective of the weight of the patient.

In one example, the cells are administered at a dose of about 200 million cells irrespective of the weight of the patient.

In one example, the cells are administered at a dose of about 300 million cells irrespective of the weight of the patient.

In some examples, cells are contained within a chamber that does not permit the cells to exit into a subject's circulation, however that permits factors secreted by the cells to enter the circulation. In this manner soluble factors may be administered to a subject by permitting the cells to secrete the factors into the subject's circulation. Such a chamber may equally be implanted at a site in a subject to increase local levels of the soluble factors, e.g., implanted in or near a pancreas.

In some examples of the disclosure, it may not be necessary or desirable to immunosuppress a patient prior to initiation of therapy with cellular compositions. Accordingly, transplantation with allogeneic, or even xenogeneic, STRO-1⁺ cells or progeny thereof may be tolerated in some instances.

However, in other instances it may be desirable or appropriate to pharmacologically immunosuppress a patient prior to initiating cell therapy and/or reduce an immune response of a subject against the cellular composition. This may be accomplished through the use of systemic or local immunosuppressive agents, or it may be accomplished by delivering the cells in an encapsulated device. The cells may be encapsulated in a capsule that is permeable to nutrients and oxygen required by the cell and therapeutic factors the cell is yet impermeable to immune humoral factors and cells. For example, the encapsulant is hypoallergenic, is easily and stably situated in a target tissue, and provides added protection to the implanted structure. These and other means for reducing or eliminating an immune response to the transplanted cells are known in the art. As an alternative, the cells may be genetically modified to reduce their immunogenicity.

### Compositions of Soluble Factors

In one example of the present disclosure, STRO-1⁺ cell-derived and/or progeny cell-derived supernatant or soluble factors are administered in the form of a composition, e.g., comprising a suitable carrier and/or excipient. For example, the carrier or excipient does not adversely affect the biological effect of the soluble factors or supernatant.

In one example, the composition comprises a composition of matter to stabilize a soluble factor or a component of supernatant, e.g., a protease inhibitor. For example, the protease inhibitor is not included in an amount sufficient to have an adverse effect on a subject.

Compositions comprising STRO-1⁺ cell-derived and/or progeny cell-derived supernatant or soluble factors may be prepared as appropriate liquid suspensions, e.g., in culture medium or in a stable carrier or a buffer solution, e.g., phosphate buffered saline. Suitable carriers are described herein above. In another example, suspensions comprising STRO-1⁺ cell-derived and/or progeny cell-derived supernatant or soluble factors are oily suspensions for injection. Suitable lipophilic solvents or vehicles include fatty oils such as sesame oil; or synthetic fatty acid esters, such as ethyl oleate or triglycerides; or liposomes. Suspensions to be used for injection may also contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Sterile injectable solutions can be prepared by incorporating the supernatant or soluble factors in the required amount in an appropriate solvent with one or a combination of ingredients described above, as required, followed by filtered sterilization.

Generally, dispersions are prepared by incorporating the supernatant or soluble factors into a sterile vehicle that contains a basic dispersion medium and the required other ingredients from those enumerated above. In the case of sterile powders for the preparation of sterile injectable solutions, exemplary methods of preparation are vacuum drying and freeze-drying which yields a powder of the active ingredient plus any additional desired ingredient from a previously sterile-filtered solution thereof. In accordance with an alternative aspect of the disclosure, the supernatant or soluble factors may be formulated with one or more additional compounds that enhance its solubility.

Other exemplary carriers or excipients are described, for example, in Hardman, et al. (2001) Goodman and Gilman's The Pharmacological Basis of Therapeutics, McGraw-Hill, New York, N. Y.; Gennaro (2000) Remington: The Science and Practice of Pharmacy, Lippincott, Williams, and Wilkins, New York, N. Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N. Y.

Therapeutic compositions typically should be sterile and stable under the conditions of manufacture and storage. The composition can be formulated as a solution, microemulsion, liposome, or other ordered structure. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be desirable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. Moreover, the soluble factors may be administered in a time release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are patented or generally known to those skilled in the art.

The supernatant or soluble factors may be administered in combination with an appropriate matrix, for instance, to provide slow release of the soluble factors.

### Additional Components of Compositions

The STRO-1⁺ cell-derived supernatant or soluble factors, STRO-1⁺ cells or progeny thereof may be administered with other beneficial drugs or biological molecules (growth factors, trophic factors). When administered with other agents, they may be administered together in a single pharmaceutical composition, or in separate pharmaceutical compositions, simultaneously or sequentially with the other agents (either before or after administration of the other agents). Bioactive factors which may be co-administered include anti-apoptotic agents (e.g., EPO, EPO mimetibody, TPO, IGF-I and IGF-II, HGF, caspase inhibitors); anti-inflammatory agents (e.g., p38 MAPK inhibitors, TGF-beta inhibitors, statins, IL-6 and IL-1 inhibitors, PEMIROLAST, TRANILAST, REMICADE, SIROLIMUS, and NSAIDs (non-steroidal anti-inflammatory drugs; e.g., TEPOXALIN, TOLMETIN, SUPROFEN); immunosupressive/immunomodulatory agents (e.g., calcineurin inhibitors, such as cyclosporine, tacrolimus; mTOR inhibitors (e.g., SIROLIMUS, EVEROLIMUS); anti-proliferatives (e.g., azathioprine, mycophenolate mofetil); corticosteroids (e.g., prednisolone, hydrocortisone); antibodies such as monoclonal anti-IL-2Ralpha receptor antibodies (e.g., basiliximab, daclizumab), polyclonal anti-T-cell antibodies (e.g., anti-thymocyte globulin (ATG); anti-lymphocyte globulin (ALG); monoclonal anti-T cell antibody OKT3)); anti-thrombogenic agents (e.g., heparin, heparin derivatives, urokinase, PPack (dextrophenylalanine proline arginine chloromethylketone), antithrombin compounds, platelet receptor antagonists, anti-thrombin antibodies, anti-platelet receptor antibodies, aspirin, dipyridamole, protamine, hirudin, prostaglandin inhibitors, and platelet inhibitors); and anti-oxidants (e.g., probucol, vitamin A, ascorbic acid, tocopherol, coenzyme Q-10, glutathione, L-cysteine, N-acetylcysteine) as well as local anesthetics.

In one example, the STRO-1⁺ cell-derived supernatant or soluble factors, STRO-1⁺ cells or progeny thereof are administered with a compound useful for treating endothelial dysfunction, such as an angiotensin converting enzyme inhibitor, peroxisome proliferator- activated receptor-activators (insulin sensitizers, e.g., the glitazones pioglitazone and rosiglitazone) and peroxisome proliferator-activated receptor- activators (fibrates, e.g., feno fibrate), antioxidants (e.g., ascorbic acid or vitamin E) or hormone replacement therapy.

In another example, a composition as described herein according to any example additionally comprises a factor that induces or enhances differentiation of a progenitor cell into a vascular cell. Exemplary factors include, vascular endothelial growth factor (VEGF), platelet derived growth factor (PDGF; e.g., PDGF-BB), and FGF.

In another example, a composition as described herein according to any example additionally comprises a tissue specific committed cell (TSCC). In this respect, International Patent Application No. PCT/AU2005/001445 demonstrates that administration of a TSCC and a STRO-1⁺ cells can lead to enhanced proliferation of the TSCC. In one example, the TSCC is a vascular cell. Administration of such a composition to a subject may lead to increased production of vasculature, e.g., leading to increased nutrients being delivered to the affected tissue.

### Medical Devices

The present disclosure also provides medical devices for use or when used in a method as described herein according to any example. For example, the present disclosure provides a syringe or catheter or other suitable delivery device comprising STRO-1⁺ cells and/or progeny cells thereof and/or soluble factors therefrom and/or a composition as described herein according to any example. Optionally, the syringe or catheter is packaged with instructions for use in a method as described herein according to any example.

In another example, the present disclosure provides an implant comprising STRO-1⁺ cells and/or progeny cells thereof and/or soluble factors therefrom and/or a composition as described herein according to any example. Optionally, the implant is packaged with instructions for use in a method as described herein according to any example. Suitable implants may be formed with a scaffold, e.g., as described herein above and STRO-1⁺ cells and/or progeny cells thereof and/or soluble factors therefrom.

### Modes of Administration

The STRO-1⁺ cell-derived supernatant or soluble factors, STRO-1⁺ cells or progeny thereof may be surgically implanted, injected, delivered (e.g., by way of a catheter or syringe), or otherwise administered systemically.

In one example, the STRO-1⁺ cell-derived supernatant or soluble factors, STRO-1⁺ cells or progeny thereof is/are delivered to the blood stream of a subject. For example, the STRO-1⁺ cell-derived supernatant or soluble factors, STRO-1⁺ cells or progeny thereof are delivered parenterally. Exemplary routes of parenteral administration include, but are not limited to, intraperitoneal, intraventricular, intracerebroventricular, intrathecal, intra-arterial, intranodal or intravenous. In one example, the STRO-1⁺ cell-derived supernatant or soluble factors, STRO-1⁺ cells or progeny thereof are delivered intra-arterially, into an aorta, into an atrium or ventricle of the heart or into a blood vessel, e.g., intravenously.

In the case of cell delivery to an atrium or ventricle of the heart, cells can be administered to the left atrium or ventricle to avoid complications that may arise from rapid delivery of cells to the lungs.

In one example, the STRO-1⁺ cell-derived supernatant or soluble factors, STRO-1⁺ cells or progeny thereof are injected into the site of delivery, e.g., using a syringe or through a catheter or a central line.

Selecting an administration regimen for a therapeutic formulation depends on several factors, including the serum or tissue turnover rate of the entity, the level of symptoms, and the immunogenicity of the entity. For example, an administration regimen maximizes the amount of therapeutic compound delivered to the patient consistent with an acceptable level of side effects. Accordingly, the amount of formulation delivered depends in part on the particular entity and the severity of the condition being treated.

In one example, STRO-1⁺ cell-derived supernatant or soluble factors, STRO-1⁺ cells or progeny thereof are delivered as a single bolus dose. Alternatively, STRO-1⁺ cell-derived supernatant or soluble factors, STRO-1⁺ cells or progeny thereof are administered by continuous infusion, or by doses at intervals of, e.g., one day, one week, or 1-7 times per week. An exemplary dose protocol is one involving the maximal dose or dose frequency that avoids significant undesirable side effects. A total weekly dose depends on the type and activity of the compound being used. Determination of the appropriate dose is made by a clinician, e.g., using parameters or factors known or suspected in the art to affect treatment or predicted to affect treatment. Generally, the dose begins with an amount somewhat less than the optimum dose and is increased by small increments thereafter until the desired or optimum effect is achieved relative to any negative side effects. Important diagnostic measures include those of symptoms of diabetes.

In some examples the cells are administered weekly, fortnightly, once every three weeks or once every four weeks.

In accordance with examples of the disclosure directed to treating or delaying the progression of an inflammatory joint disease, in one example, the STRO-1⁺ cells and/or progeny cells thereof and/or soluble factors derived therefrom are administered following diagnosis of the disorder, e.g., using standard methods known in the art and/or described herein.

### EXAMPLES

### Example 1: Immunoselection of MPCs by Selection of STRO-3⁺ Cells

Bone marrow (BM) is harvested from healthy normal adult volunteers (20-35 years old). Briefly, 40 ml of BM is aspirated from the posterior iliac crest into lithium-heparin anticoagulant-containing tubes.

BMMNC are prepared by density gradient separation using Lymphoprep™ (Nycomed Pharma, Oslo, Norway) as previously described (Zannettino, A.C. et al. (1998) Blood 92: 2613-2628). Following centrifugation at 400 x g for 30 minutes at 4°C, the buffy layer is removed with a transfer pipette and washed three times in "HHF", composed of Hank's balanced salt solution (HBSS; Life Technologies, Gaithersburg, MD), containing 5% fetal calf serum (FCS, CSL Limited, Victoria, Australia).

STRO-3⁺ (or TNAP⁺) cells were subsequently isolated by magnetic activated cell sorting as previously described (Gronthos et al. (2003) Journal of Cell Science 116: 1827-1835; Gronthos, S. and Simmons, P.J. (1995) Blood 85: 929-940). Briefly, approximately 1-3 x 10⁸ BMMNC are incubated in blocking buffer, consisting of 10% (v/v) normal rabbit serum in HHF for 20 minutes on ice. The cells are incubated with 200µl of a 10µg/ml solution of STRO-3 mAb in blocking buffer for 1 hour on ice. The cells are subsequently washed twice in HHF by centrifugation at 400 x g. A 1/50 dilution of goat anti-mouse γ-biotin (Southern Biotechnology Associates, Birmingham, UK) in HHF buffer is added and the cells incubated for 1 hour on ice. Cells are washed twice in MACS buffer (Ca²⁺ - and Mn²⁺ -free PBS supplemented with 1% BSA, 5 mM EDTA and 0.01% sodium azide) as above and resuspended in a final volume of 0.9 ml MACS buffer.

One hundred µl streptavidin microbeads (Miltenyi Biotec; Bergisch Gladbach, Germany) are added to the cell suspension and incubated on ice for 15 minutes. The cell suspension is washed twice and resuspended in 0.5 ml of MACS buffer and subsequently loaded onto a mini MACS column (MS Columns, Miltenyi Biotec), and washed three times with 0.5 ml MACS buffer to retrieve the cells which did not bind the STRO-3 mAb (deposited on 19 December 2005 with American Type Culture Collection (ATCC) under accession number PTA-7282 - see International Publication No.WO 2006/108229). After addition of a further 1 ml MACS buffer, the column is removed from the magnet and the TNAP⁺ cells are isolated by positive pressure. An aliquot of cells from each fraction can be stained with streptavidin-FITC and the purity assessed by flow cytometry.

### Example 2: Cells Selected by STRO-3 mAb are STRO-1^{bright} Cells

Experiments were designed to confirm the potential of using STRO-3 mAb as a single reagent for isolating cells STRO-1^{bright} cells.

Given that STRO-3 (IgG1) is a different isotype to that of STRO-1 (IgM), the ability of STRO-3 to identify clonogenic CFU-F was assessed by two-color FACS analysis based on its co-expression with STRO-1⁺ cells isolated using the MACS procedure (Figure 1). The dot plot histogram represents 5 x 10⁴ events collected as listmode data. The vertical and horizontal lines were set to the reactivity levels of <1.0% mean fluorescence obtained with the isotype-matched control antibodies, 1B5 (IgG) and 1A6.12 (IgM) treated under the same conditions. The results demonstrate that a minor population of STRO-1^{bright} cells co-expressed TNAP (upper right quadrant) while the remaining STRO-1⁺ cells failed to react with the STRO-3 mAb. Cells isolated by FACS from all four quadrants were subsequently assayed for the incidence of CFU-F (Table 1).

**Table 1: Enrichment of human bone marrow cells by dual-color FACS analysis based on the co-expression of the cell surface markers STRO-1 and TNAP (refer to Figure 1). FACS sorted cells were cultured under standard clonogenic conditions in alpha MEM supplemented with 20% FCS. The data represents the mean number of day 14 colony-forming cells (CFU-F) per 10⁵ cells plated ± SE (n=3 different bone marrow aspirates). These data suggest that human MPC are exclusively restricted to the TNAP positive fraction of BM which co-express the STRO-1 antigen brightly.**

| Bone Marrow Fraction | Frequency of CFU-F/10⁵ Cells | Enrichment (Fold Increase) |
|---|---|---|
| Unfractionated BMMNC | 11.0 ± 2.2 | 1.0 |
| TNAP⁺/STRO-1^{bright} | 4,511 ± 185 | 410 |
| TNAP⁺/STRO-1^{dull} | 0.0 | 0.0 |

### Example 3: Relative Gene and Surface Protein Expression of STRO-1^{dull} and STRO-1^{bright} Cells

In the first series of experiments, semi-quantitative RT-PCR analysis was employed to examine the gene expression profile of various lineage-associated genes expressed by STRO-1^{dull} or STRO-1^{bright} populations, isolated by fluorescence activated cell sorting (Figure 2A). In the second series of experiments, flow cytometry and mean channel fluorescence analysis was employed to examine the surface protein expression profile of various lineage-associated proteins expressed by STRO-1^{dull} or STRO-1^{bright} populations, isolated by fluorescence activated cell sorting.

Total cellular RNA was prepared from either 2 x 10⁶ STRO-1^{bright} or STRO-1^{dull} sorted primary cells, chondrocyte pellets and other induced cultures and lysed using RNAzolB extraction method (Biotecx Lab. Inc., Houston, TX), according to the manufacturer's recommendations. RNA isolated from each subpopulation was then used as a template for cDNA synthesis, prepared using a first-strand cDNA synthesis kit (Pharmacia Biotech, Uppsala, Sweden). The expression of various transcripts was assessed by PCR amplification, using a standard protocol as described previously (Gronthos et al., J. Bone and Min. Res. 14:48-57, 1999). Primer sets used in this study are shown in Table 2. Following amplification, each reaction mixture was analyzed by 1.5% agarose gel electrophoresis, and visualized by ethidium bromide staining. RNA integrity was assessed by the expression of GAPDH.

Relative gene expression for each cell marker was assessed with reference to the expression of the house-keeping gene, GAPDH, using ImageQant software (Figure 2B, C). In addition, dual-colour flow cytometric analysis was used to examine the protein expression profile of *ex vivo* expanded MPC based on their expression of a wider range of cell lineage-associated markers in combination with the STRO-1 antibody. A summary of the general phenotype based on the gene and protein expression of STRO-1^{dull} and STRO-1^{bri} cultured cells is presented in Table 3. The data indicate that *ex vivo* expanded STRO-1^{bright} MPC exhibit differentially higher expression of markers associated with perivascular cells, including angiopoietin-1, VCAM-1, SDF-1, IL-1_{β}, TNFα, and RANKL. Comparisons between the protein and gene expression profiles of STRO-1^{dull} and STRO-1^{bright} cultured cells are summarized in Tables 3 and 4.

Subtractive hybridization studies were also performed in order to identify genes uniquely expressed by STRO-1^{bri} cells. Briefly, STRO-1^{dull} and STRO-1^{bright} were isolated as described above (see Figure 3A). Total RNA was prepared from STRO-1^{dull} and STRO-1^{bright} cells pooled from 5 different marrow samples using the RNA STAT-60 system (TEL-TEST). First-strand synthesize was performed using the SMART cDNA synthesis kit (Clontech Laboratories). The resultant mRNA/single-stranded cDNA hybrid was amplified by long-distance PCR (Advantage 2 PCR kit; Clontech) using specific primer sites at the 3' and 5' prime ends formed during the initial RT process according to the manufacturer's specifications. Following RsaI digestion of the STRO-1^{bright} cDNA, 2 aliquots were used to ligate different specific adaptor oligonucleotides using the Clontech PCR-Select cDNA Subtraction Kit. Two rounds of subtractive hybridization were performed using STRO-1^{bright} (tester) and STRO-1^{dull} (driver) cDNA, and vice versa, according to the manufacturer's protocol. This procedure was also performed in reverse using STRO-1^{dull} tester cDNA hybridized against STRO-1^{bright} driver cDNA.

To identify genes uniquely expressed by STRO-1^{bright} population, STRO-1^{bright}-subtracted cDNA was used to construct replicate low-density microarray filters comprising 200 randomly selected bacterial clones transformed with the STRO-1^{bright} subtracted cDNAs ligated into a T/A cloning vector. The microarrays were subsequently probed with either [³²P] dCTP-labeled STRO-1^{bright} or STRO-1^{dull} subtracted cDNA (Figure 3B-C). Differential screening identified a total of 44 clones, which were highly differentially expressed between the STRO-1^{dull} and STRO-1^{bright} subpopulations. DNA sequencing of all the differentially expressed clones revealed that only 1 clone was representative of a known stromal cell mitogen; namely, platelet-derived growth factor (PDGF) (Gronthos and Simmons, Blood. 85: 929-940, 1995). Interestingly, 6 of the 44 clones were found to contain DNA inserts corresponding to the chemokine, stromal-derived factor-1 (SDF-1). The high abundance of SDF-1 transcripts in human STRO-1^{bright} cells was confirmed by semiquantitative RT-PCR of total RNA prepared from freshly sorted STRO-1^{bright}, STRO-1^{dull}, and STRO-1^{negative} bone marrow subpopulations (Figure 3D and Table 3).

**Table 2. RT-PCR primers and conditions for the specific amplification of human mRNA**

| **Target Gene** | **Sense/ Antisense (5'-3') Primer Sequences** | **Product Size** |
|---|---|---|
| **GAPDH** | CACTGACACGTTGGCAGTGG (SEQ ID NO: 1) | 417 |
| | CATGGAGAAGGCTGGGGCTC (SEQ ID NO: 2) | |
| **SDF-1** | GAGACCCGCGCTCGTCCGCC (SEQ ID NO: 3) | 364 |
| | GCTGGACTCCTACTGTAAGGG (SEQ ID NO: 4) | |
| **IL-1β** | AGGAAGATGCTGGTTCCCTCTC (SEQ ID NO: 5) | 151 |
| | CAGTTCAGTGATCGTACAGGTGC (SEQ ID NO: 6) | |
| **LT-1** | TCACTATGGAAGATCTGATTTCTTACAGT (SEQ ID NO: 7) | 380 |
| | GGTATAAATACACATGTGCTTCTAG (SEQ ID NO: 8) | |
| **TNF-α** | TCAGATCATCTTCTCGAACC (SEQ ID NO: 9) | 361 |
| | CAGATAGATGGGCTCATACC (SEQ ID NO: 10) | |
| **KDR** | TATAGATGGTGTAACCCGGA (SEQ ID NO: 11) | 450 |
| | TTTGTCACTGAGACAGCTTGG (SEQ ID NO: 12) | |
| **RANKL** | AACAGGCCTTTCAAGGAGCTG (SEQ ID NO: 13) | 538 |
| | TAAGGAGGGGTTGGAGACCTCG (SEQ ID NO: 14) | |
| **Leptin** | ATGCATTGGGAACCCTGTGC (SEQ ID NO: 15) | 492 |
| | GCACCCAGGGCTGAGGTCCA (SEQ ID NO: 16) | |
| **CBFA-1** | GTGGACGAGGCAAGAGTTTCA (SEQ ID NO: 17) | 632 |
| | TGGCAGGTAGGTGTGGTAGTG (SEQ ID NO: 18) | |
| **PPARγ2** | AACTGCGGGGAAACTTGGGAGATTCTCC (SEQ ID NO: 18) | 341 |
| | AATAATAAGGTGGAGATGCAGGCTCC (SEQ ID NO: 19) | |
| **OCN** | ATGAGAGCCCTCACACTCCTC (SEQ ID NO: 20) | 289 |
| | CGTAGAAGCGCCGATAGGC (SEQ ID NO: 21) | |
| **MyoD** | AAGCGCCATCTCTTGAGGTA (SEQ ID NO: 22) | 270 |
| | GCGAGAAACGTGAACCTAGC (SEQ ID NO: 23) | |
| **SMMHC** | CTGGGCAACGTAGTAAAACC (SEQ ID NO: 24) | 150 |
| | TATAGCTCATTGCAGCCTCG (SEQ ID NO: 25) | |
| **GFAP** | CTGTTGCCAGAGATGGAGGTT (SEQ ID NO: 26) | 370 |
| | TCATCGCTCAGGAGGTCCTT (SEQ ID NO: 27) | |
| **Nestin** | GGCAGCGTTGGAACAGAGGTTGGA (SEQ ID NO: 28) | 460 |
| | CTCTAAACTGGAGTGGTCAGGGCT (SEQ ID NO: 29) | |
| **SOX9** | CTCTGCCTGTTTGGACTTTGT (SEQ ID NO: 30) | 598 |
| | CCTTTGCTTGCCTTTTACCTC (SEQ ID NO: 31) | |
| **Collagen type X** | AGCCAGGGTTGCCAGGACCA (SEQ ID NO: 32) | 387 |
| | TTTTCCCACTCCAGGAGGGC (SEQ ID NO: 33) | |
| **Aggrecan** | CACTGTTACCGCCACTTCCC (SEQ ID NO: 34) | 184 |
| | ACCAGCGGAAGTCCCCTTCG (SEQ ID NO: 35) | |

**Table 3. Summary of the Relative Gene Expression in STRO-1^{Bright} and STRO-1^{Dull} populations. A list of genes which displayed measurable and differential expression between the STRO-1^{Bright} and STRO-1^{Dull} populations as determined by reverse transcription-PCR are presented . Values represent the relative gene expression with reference to the house-keeping gene, GAPDH.**

| | | **Gene Expression relative to GAPDH** | |
|---|---|---|---|
| **Tissue** | **Marker** | **STRO-1^{Bright}** | **STRO-1^{Dull}** |
| **Neurons** | *GFAP (Glial Fibrillary Acidic Protein)* | 0.1 | 0.7 |
| **Bone** | *OCN (Osteocalcin)* | 1.1 | 2.5 |
| | *OSX (Osterix)* | 0.4 | 1.3 |
| | *CBFA-1 (Core Factor Binding Protein-1)* | 0.3 | 0.6 |
| **Immunoregulatory** | *RANKL (Receptor Activator of Nuclear Factor κ B) SDF-1-alpha (Stromal Derived factor-1-alpha)* | 1.6 3.2 | 0.3 0.1 |
| **Fat** | *Leptin* | 3.1 | 4.2 |
| **Cardiomyocytes** | *GATA-4* | 1.1 | 2.9 |
| **Endothelial cells** | *Ang-1 (Angiopoietin-1)* | 1.5 | 0.8 |
| **Chondrocytes** | *Sox 9* | 0.3 | 1.1 |
| | *COL X (Collagen X)* | 3.5 | 2.8 |
| **Pro-inflammatory Cytokines** | *TNF-alpha (Tumor necrosis alpha)* | 1.7 | 0.9 |

To correlate protein surface expression with density of STRO-1 expression, single cell suspensions of *ex vivo* expanded cells derived bone marrow MPC were prepared by trypsin/EDTA detachment and subsequently incubated with the STRO-1 antibody in combination with antibodies identifying a wide range of cell lineage-associated markers. STRO-1 was identified using a goat anti-murine IgM-fluorescein isothiocyanate while all other markers were identified using either a goat anti-mouse or anti-rabbit IgG- phycoerythrin. For those antibodies identifying intracellular antigens, cell preparations were first labeled with the STRO-1 antibody, fixed with cold 70% ethanol to permeabilize the cellular membrane and then incubated with intracellular antigen-specific antibodies. Isotype matched control antibodies were used under identical conditions. Dual-colour flow cytometric analysis was performed using a COULTER EPICS flow cytometer and list mode data collected. The dot plots represent 5,000 listmode events indicating the level of fluorescence intensity for each lineage cell marker (y-axis) and STRO-1 (x-axis). The vertical and horizontal quadrants were established with reference to the isotype matched negative control antibodies.

**Table 4. Summary of the Relative Protein Expression in STRO-1^{Bright} and STRO-1^{Dull} populations. A list of proteins which displayed differential expression between the STRO-1^{Bri} and STRO-1^{Dull} populations as determined by flow cytometry are presented. Values represent the relative mean fluorescence intensity of staining.**

| | | **Mean Fluorescence Intensity** | |
|---|---|---|---|
| **Tissue** | **Marker** | **STRO-1^{Bright} STRO-1^{Dull}** | |
| **Neurons** | *Neurofilament* | 1.7 | 20.5 |
| **Bone** | *ALK PHOS (Alkaline Phophatase)* | 5.7 | 44.5 |
| **Immunoregulatory** | *RANKL (Receptor Activator of Nuclear Factor κ B)* | 658.5 | 31.0 |
| **Epithelial Cells** | *CytoKeratin 10*+*13* | 1.2 | 23.3 |
| | *Cytokeratin 14* | 1.8 | 8.8 |
| **Smooth Muscle** | *α-SMA (Alpha Smooth Muscle Actin)* | 318.0 | 286.0 |
| **Chondrocytes** | *Byglycan* | 84.4 | 65.9 |
| **Basal Fibroblast** | *Tenascin C* | 22.2 | 6.9 |
| **Cardiomyocyte** | *Troponin C* | 2.5 | 15.0 |

### Example 4: Stromal cells express Angiotensin I

Human stromal cell lines HS5 and HS27A were grown in RPMI-1640 supplemented with 10% Fetal Calf Serum (FCS) and Penicillin (100 U/ml and streptomycin (100 µg/mL). Primary stromal fibroblasts were cultured from bone marrow mononuclear cells (BMMNC) as previously described (Pillai et al., Blood 107: 3520-3526, 2006). Cells were stained with FITC-conjugated anti-CD146 antibody (Ebiosciences, San Diego, CA) as well as appropriate isotype control for analysis as well as cell sorting in to CD146hi and CD146 lo populations on a FACS Aria cell sorter (BD Biosciences, San Jose, CA).

For quantitative RT-PCR, total RNA was prepared using the miRNEasy kit (Qiagen, Valencia, CA) per manufacturer's instructions; on-column DNAse digestion was performed to eliminate contamination with genomic DNA. mRNA levels were quantitated by SYBR-GREEN based quantitative Real Time-Polymerase Chain Reaction (qRT-PCR) on cDNA generated by reverse transcription.

The HS27a stromal cell line seemed to share similar functional and transcriptional profiles with those reported for the CD146^{hi} stromal precursors. Thus, the expression of CD14 on this cell line was assessed. Flow-cytometric analysis of CD146 was performed on HS27a and HS5 cells and showed that CD146 was expressed strongly by HS27a, whereas HS5 did not (Figure 4A). CD146 expression was also assessed in 9 primary cultures, established from normal bone marrow. The cultures which were analyzed within the first two to three passages demonstrated variable proportions of CD146^{hi} cells (6 to 40%), an example of which is shown in Figure 4B. The CD146^{hi} and CD146^{lo} cells from primary cultures were then evaluated for expression of two genes, CXCL12 and Angiopoietin I. As shown in Figures 3C and 3D, the CD146^{hi} population has significantly higher expression of both CXCL12 and Angiopoietin I when compared to CD146^{lo} cells.

These data indicate that stromal precurscor cells that express high levels of CD146, e.g., STRO-1 expressing MPCs also express Angiopoitin I.

### Example 5: MPCs treat or prevent endothelial dysfunction

### Methods

Sheep were sensitized to Bovine type II collagen (BColl-II] by a subcutaneous injection of an emulsion of Freund's *complete* adjuvant (1mL) and BColl-II (5 mg) administered on day 0 followed by a second injection of Freund's *incomplete* adjuvant (1 mL) and BColl-II (5 mg) on day 14. On day 28, 100 µg of BColl-II in 0.5 mL isotonic saline was injected intra-articularly into the left hock joint to promote an acute arthritis accompanied by systemic inflammation. On day 29, sheep were injected intra-venously with saline (n = 8) or 150 million MPC suspended in PBS (n = 8). Blood was collected over the subsequent 13 days and plasma levels of Il-10, fibrinogen, Activin A and C-reactive protein (CRP) were determined using ELISA commercial kits. All animals were sacrificed on day 42.

Tissues were collected and stored in ice-cold modified Krebs-Henseleit solution (KHS). Second order branches of the left descending coronary artery were dissected free from the surrounding tissue under a dissecting microscope (Stemi 2000 stereomicroscope, Carl Zeiss, Gőttengen, Germany) a
length prepared for vasomotor functional study using the Mulvany-Halpern wire myograph (Danish Myo Technologies, Denmark). Briefly, vessel segments were mounted between two parallel stainless steel wires (40 µm diameter) attached to the jaws of the wire myograph.

Ovine digital arteries were obtained from the left forelimb of the same sheep. 1mm segments were taken from the first branch of the palmar digital artery, and mounted in the wire myograph as described above.

### Drugs and solutions

The modified KHS used has the following composition (mM): NaCl 118, KCl 4.57, CaCl₂ 1.27, KH₂PO₄ 1.19, MgSO₄ 1.19, NaHCO₃ 25 and glucose 5.55. All drugs were obtained from Sigma-Aldrich company Ltd, Australia. All drugs and the KHS solutions were freshly prepared on the day of the experiment, and dissolved in distilled water. All drugs were subsequently diluted in KHS.

### Isometric tension recording

The preparations were placed in a chamber containing 5 ml of KHS, maintained at 37 °C and aerated with 95% O₂ and 5% CO₂. After an equilibration period of 15 min, when the vessel segments become normalized according to the methods developed by Mulvany and Halpern Circulation Res. 41: 19-26, 1977, and the optimal internal circumference was determined during the normalization procedure. Resting tension was applied to the blood vessels according to their normalized internal circumference, corresponding to 13.3 kPa (100 mmHg) with an adjustment factor of 0.9. After the resting tension was established, vessels were submitted to an extra equilibration period of 30 min in KHS followed by a contractile response to a standard depolarizing Krebs solution (DKS; 118 mM KCl).

### Coronary arteries: bradykinin and sodium nitroprusside -induced relaxation of arteries pre-constricted with endothelin-1

Following washout and re-equilibration (15 min), two vessel segments from the same individual were incubated with KHS containing the ibuprofen (10 µM) to eliminate any prostaglandin-mediated effects of bradykinin. They were then contracted to 75% of their maximum DKS response using endothelin-1 (3x 10⁻⁸ M). Once a steady state contraction was achieved, relaxant responses were then obtained to cumulatively increasing concentrations of either the endothelium-dependant vasodilator, bradykinin (10⁻¹¹ M to 10⁻⁵ M), or the endothelium-independent vasodilator, sodium nitroprusside (10⁻¹⁰ M to 10⁻⁴ M). Only one concentration-response curve was conducted in each segment, with two adjacent segments being used in each experiment.

### Digital arteries: carbachol and sodium nitroprusside -induced relaxation of arteries pre-constricted with 5-HT

Following washout and re-equilibration (15 min), two vessel segments from the same individual were contracted to 75% of their maximum DKS response using 5-HT (3 x 10⁻⁶ M). Once a steady state contraction has been achieved, relaxant responses were then obtained to cumulatively increasing concentrations of either the endothelium-dependant vasodilator, carbachol (10⁻⁹ M to 10⁻⁴ M), or the endothelium-independent vasodilator, sodium nitroprusside (10⁻¹⁰ M to 10⁻⁴ M). Only one concentration-response curve was conducted in each segment, with two adjacent segments being used in each experiment.

### Data analysis

Tension was continuously recorded by a computerized acquisition system and results expressed as mean ± standard error of the mean (s.e.m.) of the indicated number of separate experiments (*n*), each from a separate preparation. The data was used to construct cumulative concentration-response curves (CRC), from which EC₅₀ (the agonist concentration yielding 50% of the maximum response, expressed as geometric mean and 95% confidence intervals) values and maximum responses was calculated and statistical comparisons made using one-way or two-way analysis of variance with Dunnett's post-hoc test when appropriate, by curve fitting software (GraphPad Prism 5.0).

### Results

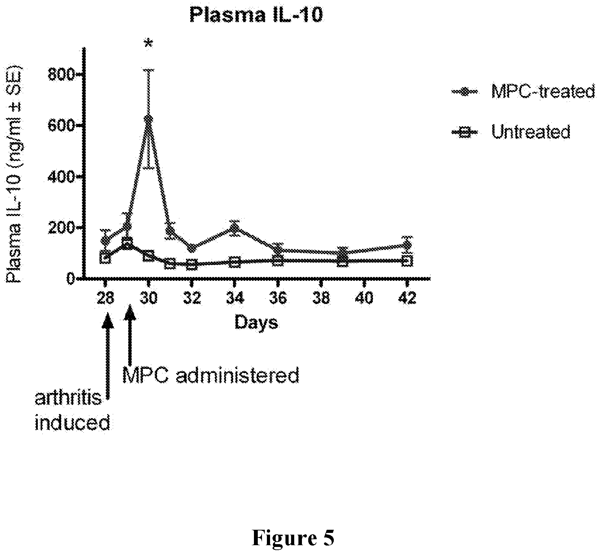

The results showed that a single IV administration of 150 million allogenic MPCs caused a spike in production of IL-10 levels on day after administration (Figure 5). IL-10 is an inflammatory cytokine which promotes circulating levels of neutrophils and helps limits their pro-inflammatory state. This in turn serves to enhance treatment of systemic infection diseases such as sepsis.

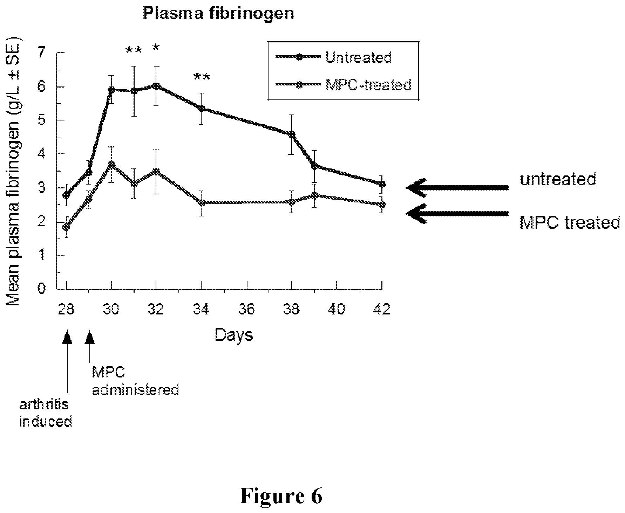

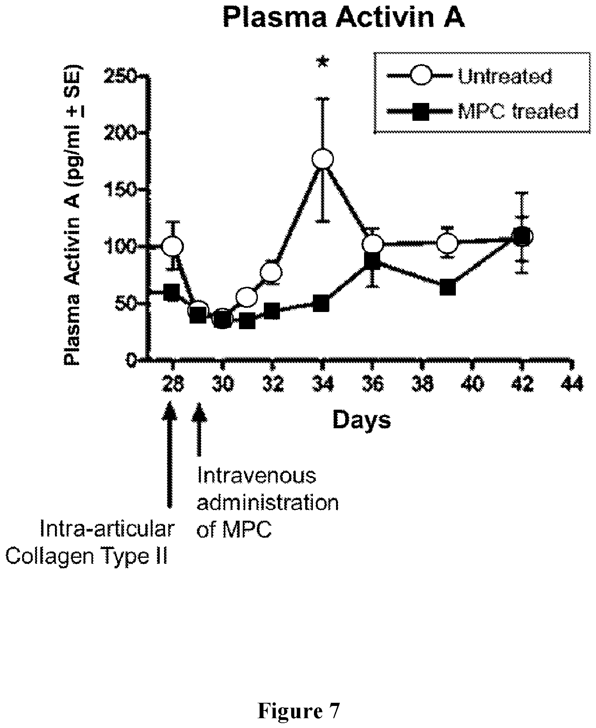

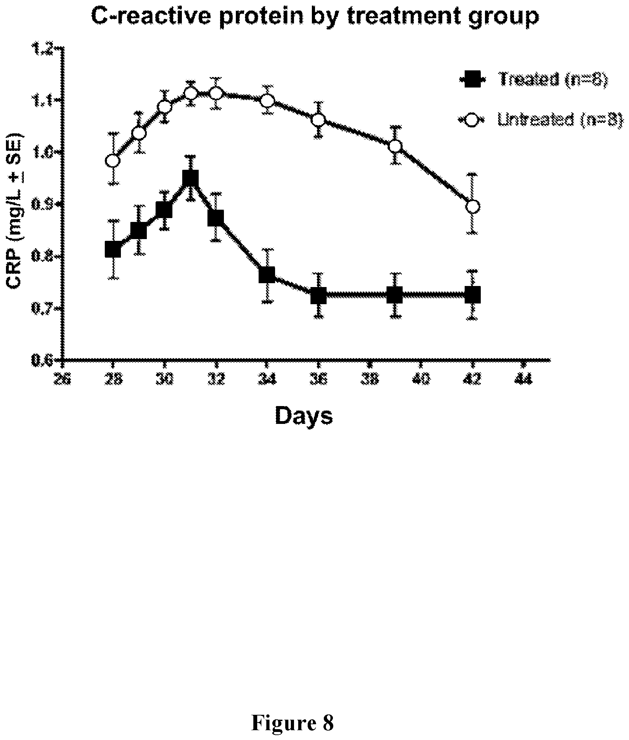

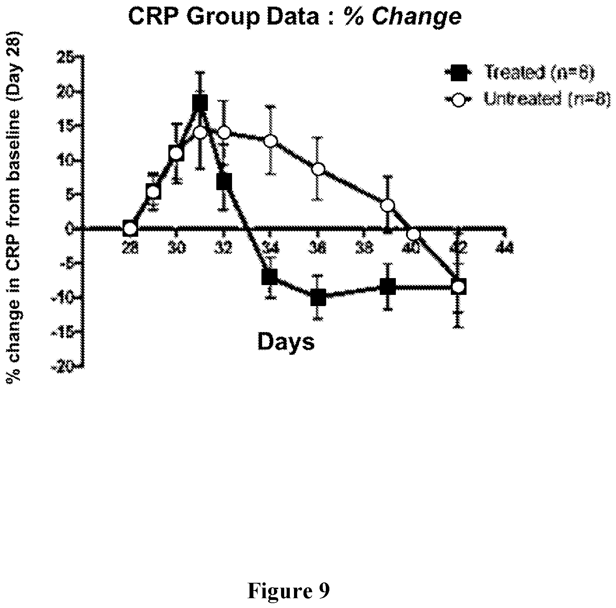

The single dose of MPCs was also effective in reducing plasma fibrinogen, Activin A and C-reactive protein relative to untreated sheep (Figures 6 - 9). Fibrinogen, Activin A and C-reactive protein are all markers of sepsis and their reduction in plasma levels is indicative of reduced symptoms of sepsis following administration of the MPCs.

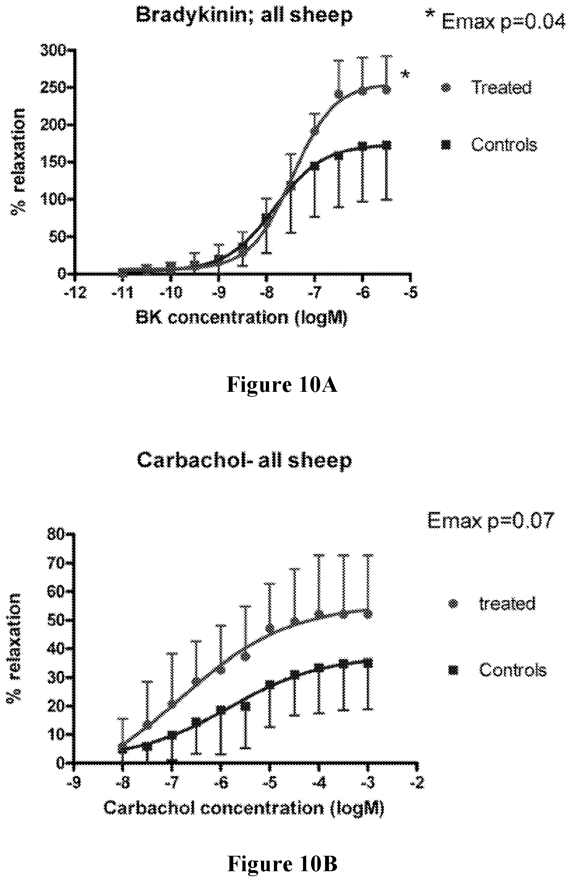

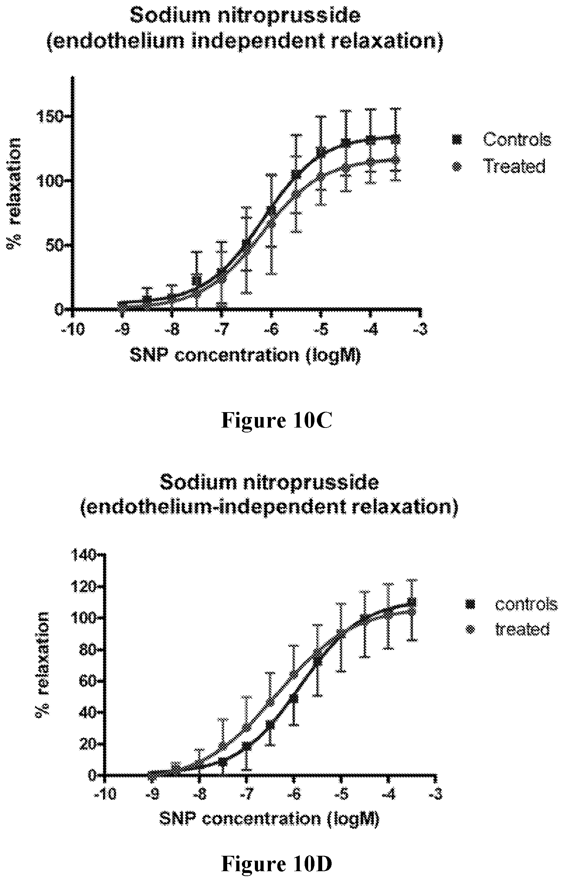

Sheep treated with MPCs demonstrated a significantly higher maximal response to carbachol or bradykinin when compared to untreated sheep (p<0.05) (Figures 10A and 10B). There was no significant difference in the response of coronary or digital arteries from the two groups to sodium nitroprusside (Figures 10C and 10D), indicating that vascular smooth muscle function was unaffected by treatment. This pre-clinical study demonstrated that MPCs when given intravenously are able to attenuate the development of systemic endothelial dysfunction.

### Example 6: MPCs in combination with PPS induces anti-inflammatory effects

### Methods

Sheep were sensitized to Bovine type II collagen (BColl-II] by a subcutaneous injection of an emulsion of Freund's *complete* adjuvant (1mL) and BColl-II (5 mg) administered on day 0 followed by a second injection of Freund's *incomplete* adjuvant (1 mL) and BColl-II (5 mg) on day 14. On day 28, 100 µg of BColl-II in 0.5 mL isotonic saline was injected intra-articularly into the left hock joint to promote an acute arthritis accompanied by systemic inflammation. On day 29, sheep were injected intra-venously with 75 million MPC suspended in PBS (n = 6) or 75 million MPC suspended in PBS plus 75 milligrams Pentosan Polysulfate (PPS) (n = 6) or 75 milligrams PPS. Blood was collected over the subsequent 13 days and plasma levels of Il-10, fibrinogen, Activin A and C-reactive protein (CRP) were determined using ELISA commercial kits. All animals were sacrificed on day 42.

Ovine digital and coronary arteries were obtained as detailed above. Furthermore, isometric tension recording, data analysis and digital and coronary artery function analysis was conducted as described above.

### Results

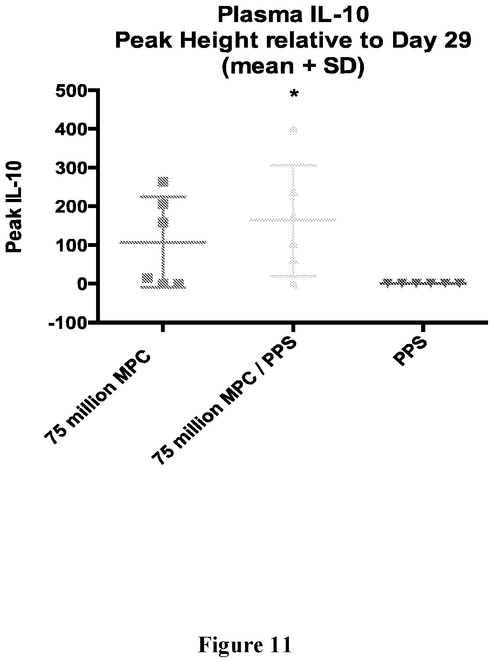

The results show that a single IV administration of 75 million allogenic MPCs plus PPS caused a significant elevation (relative to baseline) in production of plasma IL-10 levels compared with PPS alone (Figure 11). IL-10 is an inflammatory cytokine which promotes circulating levels of neutrophils and helps limits their pro-inflammatory state. This in turn serves to enhance treatment of systemic infection diseases such as sepsis.

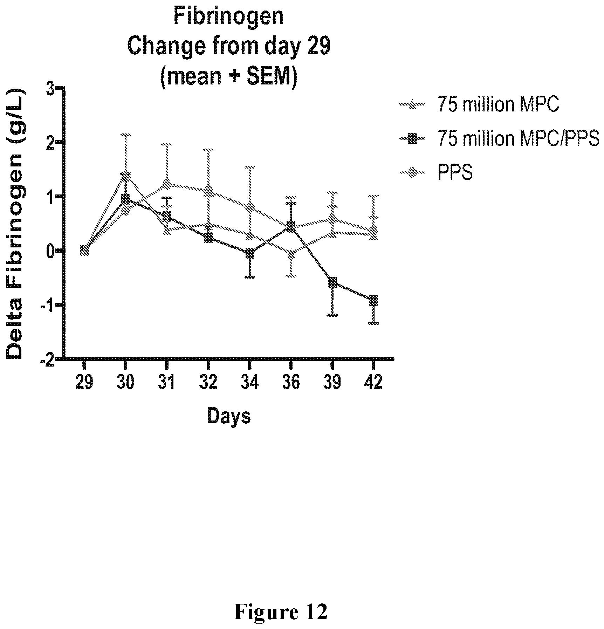

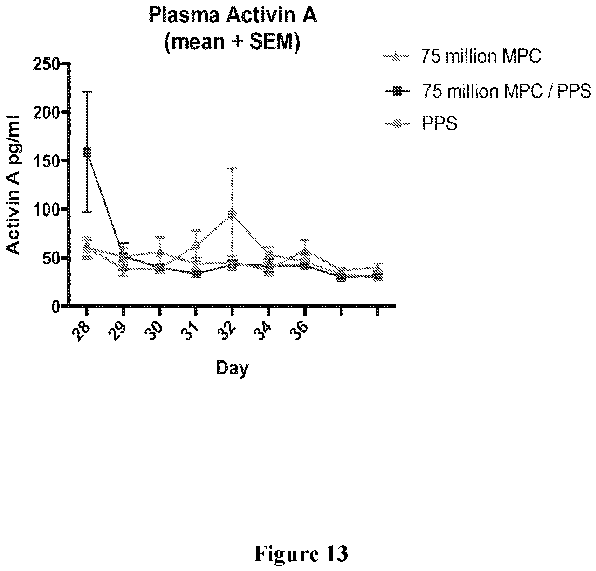

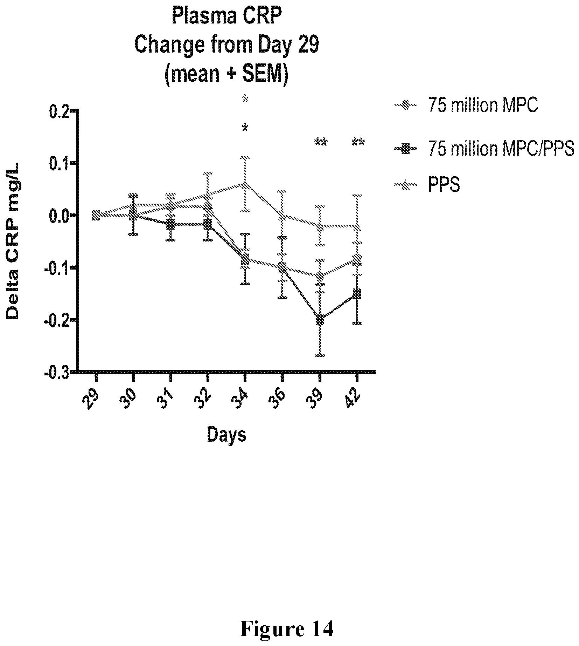

The single dose of MPCs plus PPS was effective in reducing plasma fibrinogen compared with MPCs or PPS alone from day 36 (Figure 12). Activin A protein levels in both of the MPC treated groups (75 million MPC ± PPS) were reduced relative to the PPS treated controls (Figure 13 and 14). C-reactive protein levels relative to baseline were reduced in both of the MPC treated groups (75 million MPC ± PPS) but not PPS treated controls. Fibrinogen, Activin A and C-reactive protein are all markers of sepsis and their reduction in plasma levels is indicative of reduced symptoms of sepsis following administration of the MPCs ± PPS.

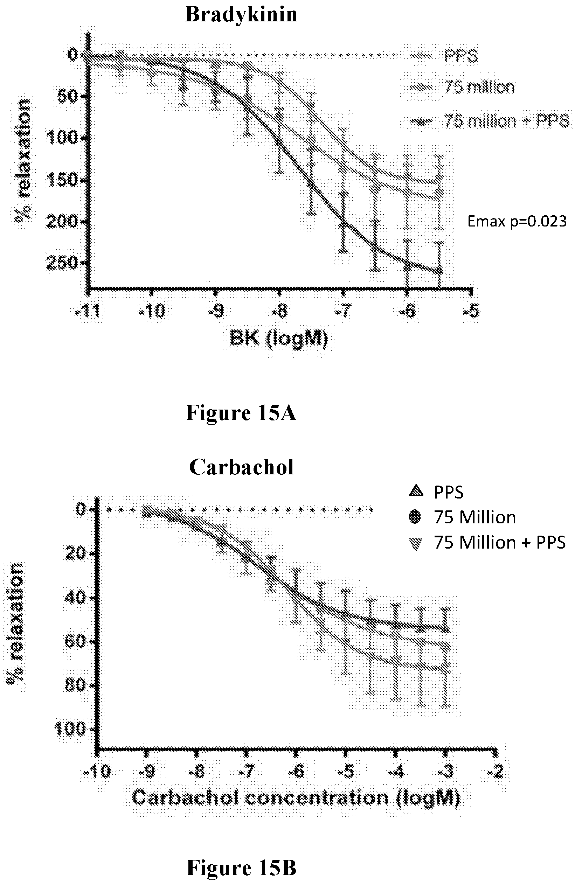

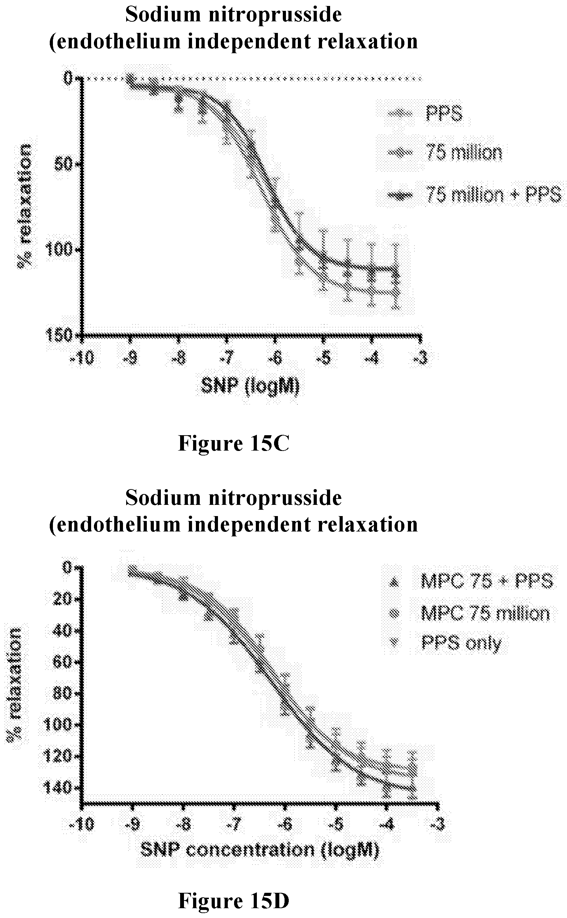

Sheep treated with MPCs plus PPS demonstrated a significantly higher maximal response of coronary arteries to bradykinin when compared to sheep treated with PPS alone (p<0.05) (Figures 15A), indicating a direct beneficial effect on the vascular endothelium. Sheep treated with MPCs plus PPS demonstrated an improvement in the maximum endothelium-dependent relaxation response in digital arteries to carbachol compared to sheep treated with PPS alone (Figure 15B). There was no significant difference in the response of coronary or digital arteries from the two groups to sodium nitroprusside (Figures 15C and 15D) indicating that vascular smooth muscle function was unaffected by treatment.

## Claims

1. A population of cells enriched for STRO-1^{bright} cells for use in treating or preventing diabetic nephropathy in a human subject, wherein the population of cells comprises at least 0.1% STRO-1^{bright} cells and is formulated for systemic administration.

2. The population of cells for the use according to claim 1, wherein the population of cells is formulated to be administered intravenously.

3. The population of cells for the use according to claim 1 or 2, wherein the population of cells is formulated to be administered at a dose of between 0.1 x 10⁶ to 5 x 10⁶ STRO-1^{bright} cells per kilogram.

4. The population of cells for the use according to any one of claims 1 to 3, wherein the population of cells is formulated to be administered at a constant body dose, such as a dose of about 100 million to 300 million cells irrespective of the weight of the patient or a dose of about 150 million cells irrespective of the weight of the patient.

5. The population of cells for the use according to any one of claims 1 to 4, wherein the population of cells is to be administered once weekly or less often.

6. The population of cells for the use according to any one of claims 1 to 5, wherein the population of cells is autogeneic or allogeneic.

7. The population of cells for the use according to any one of claims 1 to 6, wherein the population of cells has been culture expanded prior to the use.

8. The population of cells for the use according to any one of claims 1 to 7, wherein the population of cells expresses tissue non-specific alkaline phosphatase (TNAP).

9. The population of cells for the use according to any one of claims 1 to 8, wherein the population of cells is administered in combination with pentosan polysulfate (PPS) or a pharmaceutically acceptable salt thereof.

10. The population of cells for the use according to claim 9, wherein the pharmaceutically acceptable salt of pentosan polysulfate is the sodium salt.

11. The population of cells for the use according to claim 9, wherein the pentosan polysulfate or pharmaceutically acceptable salt thereof is administered at a dose of about 1 mg to 100 mg.

## Patentansprüche

1. Zellpopulation, die mit STRO-1^{bright}-Zellen angereichert ist, zur Verwendung in der Behandlung oder Verhinderung von diabetischer Nephropathie in einem menschlichen Lebewesen, wobei die Zellpopulation mindestens 0,1% STRO-1^{bright}-Zellen umfasst und für systemische Verabreichung formuliert ist.

2. Zellpopulation zur Verwendung gemäß Anspruch 1, wobei die Zellpopulation dazu formuliert ist, intravenös verabreicht zu werden.

3. Zellpopulation zur Verwendung gemäß Anspruch 1 oder 2, wobei die Zellpopulation dazu formuliert ist, in einer Dosis zwischen 0,1 x 10⁶ bis 5 x 10⁶ STRO-1^{bright}-Zellen pro Kilogramm verabreicht zu werden.

4. Zellpopulation zur Verwendung gemäß einem der Ansprüche 1 bis 3, wobei die Zellpopulation dazu formuliert ist, in einer konstanten Körperdosis, wie einer Dosis von etwa 100 Millionen bis 300 Millionen Zellen unabhängig vom Gewicht des Patienten oder einer Dosis von etwa 150 Millionen Zellen unabhängig vom Gewicht des Patienten, verabreicht zu werden.

5. Zellpopulation zur Verwendung gemäß einem der Ansprüche 1 bis 4, wobei die Zellpopulation einmal wöchentlich oder weniger oft zu verabreichen ist.

6. Zellpopulation zur Verwendung gemäß einem der Ansprüche 1 bis 5, wobei die Zellpopulation autogen oder allogen ist.

7. Zellpopulation zur Verwendung gemäß einem der Ansprüche 1 bis 6, wobei die Zellpopulation vor der Verwendung in Kultur vermehrt wurde.

8. Zellpopulation zur Verwendung gemäß einem der Ansprüche 1 bis 7, wobei die Zellpopulation gewebeunspezifische alkalische Phosphatase (tissue non-specific alkaline phosphatase; TNAP) exprimiert.

9. Zellpopulation zur Verwendung gemäß einem der Ansprüche 1 bis 8, wobei die Zellpopulation in Kombination mit Pentosanpolysulfat (PPS) oder einem pharmazeutisch verträglichen Salz davon verabreicht wird.

10. Zellpopulation zur Verwendung gemäß Anspruch 9, wobei das pharmazeutisch verträgliche Salz von Pentosanpolysulfat das Natriumsalz ist.

11. Zellpopulation zur Verwendung gemäß Anspruch 9, wobei das Pentosanpolysulfat oder pharmazeutisch verträgliche Salz davon in einer Dosis von etwa 1 mg bis 100 mg verabreicht wird.

## Revendications

1. Population de cellules enrichies en cellules STRO-1^{bright} destinée à être utilisée dans le traitement ou la prévention de la néphropathie diabétique chez un sujet humain, où la population de cellules comprend au moins 0,1 % de cellules STRO-1^{bright} et est formulée pour une administration systémique.

2. Population de cellules destinée à être utilisée selon la revendication 1, où la population de cellules est formulée pour être administrée par voie intraveineuse.

3. Population de cellules destinée à être utilisée selon la revendication 1 ou 2, où la population de cellules est formulée pour être administrée à une dose comprise entre 0,1 x 10⁶ et 5 x 10⁶ cellules STRO-1^{bright} par kilogramme.

4. Population de cellules destinée à être utilisée selon l'une quelconque des revendications 1 à 3, où la population de cellules est formulée pour être administrée à une dose corporelle constante, comme une dose d'environ 100 millions à 300 millions de cellules sans tenir compte du poids du patient ou à une dose d'environ 150 millions de cellules sans tenir compte du poids du patient.

5. Population de cellules destinée à être utilisée selon l'une quelconque des revendications 1 à 4, où la population de cellules doit être administrée une fois par semaine ou moins fréquemment.

6. Population de cellules destinée à être utilisée selon l'une quelconque des revendications 1 à 5, où la population de cellules est autogène ou allogénique.

7. Population de cellules destinée à être utilisée selon l'une quelconque des revendications 1 à 6, où la population de cellules a été soumise à une expansion en culture avant l'utilisation.

8. Population de cellules destinée à être utilisée selon l'une quelconque des revendications 1 à 7, où la population de cellules exprime la phosphatase alcaline non tissu-spécifique (TNAP).

9. Population de cellules destinée à être utilisée selon l'une quelconque des revendications 1 à 8, où la population de cellules est administrée en combinaison avec du pentosane polysulfate (PPS) ou un sel de celui-ci pharmaceutiquement acceptable.

10. Population de cellules destinée à être utilisée selon la revendication 9, dans laquelle le sel de pentosane polysulfate pharmaceutiquement acceptable est le sel de sodium.

11. Population de cellules destinée à être utilisée selon la revendication 9, dans laquelle le pentosane polysulfate ou sel de celui-ci pharmaceutiquement acceptable est administré à une dose d'environ 1 mg à 100 mg.
